(19) Europäisches Patentamt
European Patent Office
Office européen des brevets

(11) EP 0 707 711 B1

(12) **EUROPEAN PATENT SPECIFICATION**

(45) Date of publication and mention of the grant of the patent:
**14.04.1999 Bulletin 1999/15**

(21) Application number: **94922537.9**

(22) Date of filing: **13.07.1994**

(51) Int Cl.$^{6}$: **G01N 33/92**, G01N 21/19, G01N 21/25, G01N 21/64

(86) International application number:
**PCT/US94/07836**

(87) International publication number:
**WO 95/02826 (26.01.1995 Gazette 1995/05)**

(54) **DIRECT CHOLESTEROL ASSAY**

DIREKTER CHOLESTEROLASSAY

DOSAGE DIRECT DU CHOLESTEROL

(84) Designated Contracting States:
**AT BE CH DE DK ES FR GB GR IE IT LI NL SE**

(30) Priority: **14.07.1993 US 91499**

(43) Date of publication of application:
**24.04.1996 Bulletin 1996/17**

(73) Proprietor: **RESEARCH CORPORATION TECHNOLOGIES, INC.**
**Tucson, Arizona 85711-3335 (US)**

(72) Inventor: **PURDIE, Neil**
**Stillwater, OK 74074 (US)**

(74) Representative: **Körber, Wolfhart, Dr. rer.nat.**
**Patentanwälte**
**Mitscherlich & Partner,**
**Sonnenstrasse 33**
**80331 München (DE)**

(56) References cited:
**WO-A-91/10892**

- **ANAL. CHEM. (1991), 63(24), 2947-51 CODEN: ANCHAM;ISSN: 0003-2700 PURDIE, NEIL ET AL 'Direct measure of the low-density fractions of serum cholesterol'**
- **CANADIAN JOURNAL OF CHEMISTRY, vol.29, 1951, OTTAWA, CA pages 217 - 222 R.H. COX AND E.Y. SPENCER 'The estimation of some 17-alkyl-substituted steroids with the zinc chloride-acetyl chloride (Tshugaev) reagent and a postulated reaction mechanism' cited in the application**
- **CHEMICAL ABSTRACTS, vol. 84, no. 19, 10 May 1976, Columbus, Ohio, US; abstract no. 132218, KHOLODOVA, YU.D. ET AL. 'Chugaev's reaction for sterols' page 211 ;**
- **CHEMICAL ABSTRACTS, vol. 108, no. 24, 13 June 1988, Columbus, Ohio, US; abstract no. 210073, KHOLODOVA, YU.D. 'Spectral properties of steroid hormones, sapogenins and alkaloids in the Chugaev reaction as a function of their structure' page 357 ;**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 707 711 B1

Printed by Jouve, 75001 PARIS (FR)

## Description

The present invention is related to a method of forming a spectrophotometrically active cholesterol product that is particularly utile in clinical detection methods for cholesterol using, for example, fluorescence spectrophotometry, derivative absorption spectrophotometry, circular dichroism and more especially absorption spectrophotometry. More specifically, the present invention permits the direct, simultaneous quantitative determination of total cholesterol and cholesterol subfractions in clinical samples. The present invention also relates to a chemical reagent system useful for forming spectrophotometrically active products with analytes such as cholesterol and to a spectrophotometer apparatus useful in the aforesaid absorption detection chemical methods.

Although cholesterol levels are widely regarded as a reliable indicator of prospective health problems attributable, for example, to coronary disease, these levels are not easily measured by routine analytical methods involving spectrophotometry since cholesterol is not a colored material. Thus previously known methods for cholesterol determination generally require a color derivatization step. Examples of these include the Liebermann-Burchard reaction, the Zak reaction and the Abell-Kendall reaction, all of which occur in nonaqueous media and require special control of reaction conditions. Another reaction known in this regard is the oxidizing enzyme-dye reaction.

The color derivatization steps known heretofore, however, do not provide a derivative of the cholesterol molecule itself; rather, they provide secondary products of cholesterol oxidation which means that the spectrophotometric measurement of cholesterol is an indirect one. That is, the intensity of the color is not a direct measure of cholesterol concentration.

As noted hereinbefore, most routine analytical methods for practical purposes employ spectrophotometry. Spectrophotometry refers to the measurement of the absorption or transmission of incident light through solutions of test compounds. Typically, compounds of interest have characteristic spectra, transmitting or absorbing specific wavelengths of light, which can be used to determine the presence of these compounds or measure their concentration in test samples. Instruments designed for spectrophotometric absorption have a light source, for which the emitted wavelength is known and may be adjusted, and one or more detectors sensitive to desired wavelengths of transmitted or reflected light. Spectrophotometric absorption can be used to determine the amount of a given compound that is present in a test sample.

Circular dichroism (CD) is a special type of absorption method in which the molecular composition of an analyte results in differential absorption of incident light not only at a specific wavelength but also of a particular polarization state. Circular dichroism is a chiroptical method which allows one to differentiate between different enantiomers; that is, optical isomers having one or more asymmetric carbon atom (chiral) centers. When utilizing CD, generally a sample is illuminated by two circularly polarized beams of light traveling in unison. Both beams pass through the sample simultaneously and are absorbed. If the sample is optically active, the beams are absorbed to different extents. The differences in absorption of the beams can then be displayed as a function of the wavelength of the incident light beam as a CD spectrum. No difference in absorption is observed for optically inactive absorbers so that these compounds are not detected by a CD detecting system. The use of CD as a chiroptical method has been fully described in scientific literature, such as Lambert, J. B. et al. "Organic Structural Analysis", Macmillan, New York, NY 1976.

Early applications of the CD method dealt primarily dealt with elucidation of molecular structures, especially natural products for which a technique capable of confirming or establishing absolute stereochemistry was critical. However, CD has also reportedly been used in a clinical method to quantitatively determine unconjugated bilirubin in blood plasma, Grahnen, A. et al. Clinica Chimica Acta, 52, 187-196 (1974). In the method thus disclosed, a complex was formed between bilirubin and human serum albumin as a CD probe for bilirubin analysis.

Clinical applications of circular dichroism are also discussed by Neil Purdie and Kathy A. Swallows in Analytical Chemistry, Vol. 61, No. 2, pp. 77A-89A (1989). Possible clinical applications of CD are disclosed to include measurement of cholesterol levels and detection of anabolic steroids. However, suitable chemical reagents for carrying out such testing are not disclosed.

Regarding the use of spectrophotometric absorption, fluorescence, derivative spectrophotometry or CD methods herein disclosed to measure cholesterol levels, it is noted that the population at large is continually advised that it is prudent to know serum cholesterol levels and constantly reminded that an uncontrolled diet and a lack of exercise can lead to accumulation of arterial plaque that will increase the risk of atherosclerosis and coronary heart disease. Statistical studies, such as those reported by Kannel, W. B. et al. in "Serum Cholesterol, Lipoproteins and the Risk of Coronary Heart Disease: The Framingham Study" Ann. Intern. Med., 74:1-11 (1971) and Castelli, W. P. et al. in "Incidence of Coronary Heart Disease and Lipoprotein Cholesterol Levels", JAMA, 256:2835-2838 (1986), have shown that other risk factors, such as age, gender, heredity, tobacco, alcohol consumption etc. must also be considered when counselling patients about the risks.

The magnitude of the program for screening the general public is so immense that automated methods for cholesterol determinations are necessary. The tests currently used differ in complexity from the simple dip-stick approach, which uses a color sensitive reaction on a paper support, to sophisticated lipid profile tests in which the distribution of cholesterol among the various solubilizing molecular species is determined, Abbott, R. D. et al. "Joint Distribution of Lipoprotein Cholesterol Classes, The Framingham Study, Arteriosclerosis, 3:260-272 (1983). Here, the dipstick approach is only a preliminary qualitative test upon which a decision for the fuller, more quantitative measurement can be based.

At the conclusion of a recent extensive study of how health risk factors are related to elevated levels of serum cholesterol, a report entitled "Current Status of Blood Cholesterol Measurement in Clinical Laboratories of the United States, A Report from the Laboratory Standardization Panel of the National Cholesterol Education Program", Clin. Chem., 34:193-201 (1988), was prepared by the Laboratory Standardization Panel (LSP) of the National Cholesterol Education Program (NCEP). In this report, the measure or risk was correlated with three ranges of total cholesterol (TC): low risk if less than 200 mg/dL; moderate risk in the range 200-239 mg/dL; and high risk if greater than 240 mg/dL. In order to place a particular individual into one or another of these categories, all that is required is a serum TC measurement. The other risk factors, such as those identified by Kannel et al. and Castelli et al., supra, are then added as a basis for further patient counselling. This relatively simple approach replaces an earlier recommendation in Kannel et al., supra, and in Superko, H. R. et al. "High-Density Lipoprotein Cholesterol Measurements - A Help or Hinderance in Practical Clinical Medicine", JAMA 256:2714-2717 (1986) in which relative risk was established using a ratio of TC to high density lipoprotein cholesterol (HDL-C) equal to 5. A ratio lower than 5 implies a high level of HDL-C and a low relative risk. For this diagnosis, HDL-C is measured in a second independent test.

The same report by the LSP hastened to add that there were serious inaccuracies in measurements made by numerous clinical laboratories in the determination of the amount of TC present in human serum reference standards.

Statistically the results showed that, in data from 1500 laboratories, 47% failed to measure the true value to within a coefficient of variance (CV) of ± 5%, and 18% of these failed at a CV of ± 10%. As a consequence, the LSP recommended that an improvement in CV to within ± 3% for TC should be achieved by 1992. Recent surveys indicate that certified laboratories are well on their way to meeting that challenge, using the current clinical methods and instrumentation, as reported, e.g. in Posnick, L. "Labs now Better at Cholesterol Tests Data Show", Clin. Chem. News 15 (9):14 (1989). The LSP did not report the inaccuracies associated with the determination of the distribution of cholesterol among the various lipids and lipoproteins, but did indicate that an evaluation would be made in the future. The very poor proficiency and lack of reliability in the measurement of serum or plasma HDL-C, has been eloquently described in Superko, H. R. et al., supra, and in Warnick, G. R. et al. "HDL Cholesterol: Results of Interlaboratory Proficiency Test" Clin. Chem. 26:169-170 (1980) and Grundy, S. M. et al. "The Place of HDL in Cholesterol Management. A Perspective from the National Cholesterol Education Program" Arch. Inter. Med. 149:505-510 (1989) where interlaboratory CV's as high as 38% were reported. A 1987 evaluation by the College of American Pathologists (CAP) of the measurement of the same sample for HDL-C by over two thousand laboratories showed that more than one third differed by more than 5% from the reference value. Interlaboratory CV's amount groups using the same method did improve to 16.5%, but it is still too imprecise to be of any predictive clinical value. For this reason, the TC:HDL-C ratio is no longer used in risk assessment, although it offers potential advantages in defining the true clinical picture.

Regarding presently used lipid profile studies, cholesterol is known to be distributed in the serum mainly associated with high density lipoprotein (HDL-C) and low density lipoprotein (LDL-C) fractions and with triglycerides as the very low density lipoprotein cholesterol (VLDL-C) fraction. There is plenty of statistical evidence from a number of long term clinical tests to indicate that a high proportion of HDL-C and a low proportion of LDL-C is associated with lower relative risk or in simpler terms, high levels of LDL-C are to be avoided where possible. HDL-C is beneficial, provided the level is not excessively low, i.e., less than 30 mg/dL. VLDL-C cholesterol has not been implicated in any risk determination, but high triglyceride itself can be a serious problem.

In a typical lipid profile study, total cholesterols are measured directly and HDL-C is measured in the supernatant remaining after treatment of the sample with an agent to precipitate out LDL-C and VLDL-C. VLDL-C is taken to be a fixed fraction (e.g., 0.2) of the triglyceride, which is also measured directly in a separate assay. LDL-C is calculated from these figures and is not measured directly. The propagation of errors in each of the three independent measurements make LDL-C the fraction known with least overall accuracy and precision, although it may be the most significant aspect of cardiovascular risk. Because of this, it is difficult to meaningfully monitor and establish that clinical progress has been made in LDL-C reduction therapy with time.

At a workshop and subsequent roundtable session held at the 43rd Meeting of the American Association for Clinical Chemistry, the present state of the art in this area was summarized, as reported in Baillie, E. G. et al. "Standardization and Clinical Utility of Lipid Determinations", Workshop, 43rd National Meeting American Association for Clinical Chemistry, 1991 and Warnick, G. R. "Standardization of HDL Cholesterol Measurement" Roundtable, 43rd National Meeting, American Association for Clinical Chemistry, 1991. From these proceedings, it was concluded that accuracy is essential in HDL-C measurement. While presently available precipitation methods can give satisfactory results, the values obtained by these methods in routine clinical laboratory settings do not meet real medical needs. The CAP comprehensive chemistry proficiency survey from 1982 to 1991 for HDL-C showed interlaboratory CVs of about 20% in 1991, with no overall improvement since 1982. The CVs delivered by clinical instruments used for HDL-C measurements ranged from 7.6% for the Dimension to 50% for the Ektachem.

At the sessions, it was also noted that direct methods for LDL cholesterol are needed. The use of triglyceride determinations to estimate VLDL-C by the Friedewald equation is, at present, the method of choice. To quote the workshop syllabus, "The variability typically observed in the measurement of total and HDL cholesterol and triglycerides may preclude attaining acceptable precision. In fact, to achieve the ideal precision in LDL cholesterol estimation, the precision of the constituent measurements must be better than their ideal specifications."

In WO 91/10892 there is discussed a method of contacting cholesterol with Chugaev reagents, particularly $ZnCl_2$ in glacial acetic acid and acetyl chloride. In performing the desired reaction the use of sodium perchlorate is suggested.

Neil Purdie et al. discuss in Anal. Chem. 63 (1991), 2947-2951 the direct measure of low-density fractions of serum cholesterol wherein a coloured product from a Chuagev reaction with cholesterol is reported.

It is thus established that there is a need for a relatively simple, reliable and repeatable assay method to directly and simultaneously determine the amount of cholesterol, both total cholesterol and its distribution among the various subfractions without the need for precipitation or separate measurements of these subfractions.

The present invention provides a method of forming a spectrophotometrically active cholesterol product which can be employed in an assay for the direct, simultaneous, quantitative determination of total cholesterol and its distribution among HDL-C, LDL-C and VLDL-C subfractions. The spectrophotometric activity of the product may be measured by conventional absorption, fluorescence, first and second derivative spectrophotometrics, as well as circular dichroism.

Thus, in one embodiment the present invention provides a method for forming a spectrophotometrically active product of cholesterol which comprises contacting cholesterol with an acyl compound having the formula:

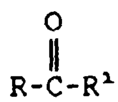

wherein $R^1$ is halogen, R is lower alkyl and a perchlorate effective to form a spectrophotometrically active product of said cholesterol, said perchlorate selected from the group consisting of zinc perchlorate, barium perchlorate and perchloric acid.

When formed in a test sample, for example, the spectrophotometric activity may be evaluated to determine the amount of cholesterol present in the sample, including its distribution among HDL-C, LDL-C and VLDL-C subfractions. The method of this embodiment permits a quick and repeatable method for the direct, simultaneous quantitative determination of cholesterol, both total cholesterol and subfraction distribution, at ambient temperature, without the need for precipitation or separate subfraction measurement.

In another embodiment the present invention provides for a clinical method for determining the amount of cholesterol (in cholesterol subfractions) in a test sample which comprises contacting the test sample in which cholesterol is present with an acyl compound having the formula:

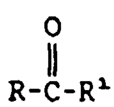

wherein $R^1$ is halogen, R is lower alkyl and a perchlorate effective to form a spectrophotometrically active product with said cholesterol, said perchlorate selected from the group consisting of zinc perchlorate, barium perchlorate and perchloric acid, and evaluating said spectrophotometric activity to determine the amoutn of said cholesterol present in said sample. In particular the method comprises forming a reaction product with the cholesterol and then either performing step ($a^1$), ($a^2$), ($a^3$) or ($a^4$), wherein steps ($a^3$) and ($a^4$) may be followed by calculation of cholesterol concentrations using matrix mathematics and constants derived for the particular cholesterol subfraction analyzed:

Step ($a^1$) determining the CD absorption spectrum of the test sample over a range from 150 to 700 nm (preferably from 360 nm to 700 nm);

Step ($a^2$) determining the CD absorption of the test sample at one or more discrete wavelengths within a range from 150 nm to 700 nm (preferably from 360 nm to 700 nm);

Step ($a^3$) determining the spectrophotometric, fluorescence or derivative spectrophotometric absorption of the test sample at three or more discrete wavelengths within a range from 150 nm to 700 nm (preferably 360 nm to 700 nm).

Step ($a^4$) determining the spectrophotometric fluorescence or derivative spectrophotometric absorption of the test sample over a range from 150 nm to 700 nm (preferably from 360 nm to 700 nm).

The invention further provides novel absorption detection apparatuses for practicing certain of the present inventive methods, which apparatuses are exemplified, but not limited, by the following:

A spectrophotometric absorption instrument for determining the amount of VLDL-C, LDL-C, HDL-C and TC present in a test sample, the instrument comprising means for determining the spectrophotometric absorption spectrum of the test sample at 3 or more distinct wavelengths, within the range of 150 to 700 nm (preferably 360 nm-700 nm), and means for determining the amount of VLDL-C, LDL-C, HDL-C and TC present in the test sample based on, for example, the spectrophotometric absorption of the cholesterol reaction products in the test sample. Optionally, the instrument further comprises means for adding reagent to the test sample.

The present invention will become more fully understood from the detailed description given here and below and the accompanying drawings which are given by way of illustration only, and thus, are not limitative of the present invention.

Figure 1 is a full CD spectrum for the optically active colored product obtained from the reaction of Chugaev reagents with cholesterol. Curve (a) is representative of the total cholesterol, while the shaded area is the spectrum after the addition of the LDL-C, VLDL-C precipitating agent and is therefore representative of the HDL-C fraction only.

Figure 2 represents the correlation between TC as measured in serum samples processed by two different labs using prior art processes (Labs A and B), versus total cholesterol as measured by the CD method of the present invention (this lab); y = -10.209 + 1.0055x, $R^2$ = 0.835.

Figure 3(a) is a graph of TC vs. (VLDL + LDL)-C using a CD method of the present invention (this lab); y = 5.0554 + 0.84693x, $R^2$ = 0.932.

Figure 3(b) is a graph of TC vs. (VLDL + LDL)-C using a prior art process (LAB-A); y = -47.672 + 0.98751x, $R^2$ = 0.987.

Figure 3(c) is a graph of TC vs. (VLDL + LDL)-C using a prior art process (LAB-B); y = 046.5222 + 0.9869x, $R^2$ = 0.98.

Figure 4(a) is a graph of TC vs. HDL-C using the CD method of the present invention (this lab); y - 5.2861 + 0.14995x, $R^2$ = 0.335.

Figure 4(b) is a graph of TC vs. HDL-C using a prior art process (LAB-A) ; y = 47.648 + 0.012569x, $R^2$ = 0.001.

Figure 4(c) is a graph of TC vs. HDL-C using a prior art process (LAB-B); y = 46.522 + 0.0131x, $R^2$ = 0.06.

Figure 5 is a schematic of a CD, wherein:

LS is the high intensity conventional light source or laser source; M1 and M2 are monochromators required for full spectral data; P is the linearly polarizing element; Q is the circularly polarizing element; S is the sample cell; D is the detector (of which there may be up to three); and REC is the recorder.

Figure 6 is a graph of the absorption spectrum of whole serum over the wavelength range of 400 nm-700 nm.

Figure 7(a) is a graph of the fluorescence spectrum for VLDC-C subfraction;

Figure 7(b) is a graph of the fluorescence spectrum for LDL-C subfraction;

Figure 7(c) is a graph of the fluorescence spectrum of HDL-C subfraction;

Figure 8(a) is a graph of the first (solid line) and second (dotted line) derivative of the conventional absorbance spectrum of the VLDL-C subfraction (sigma);

Figure 8(b) is a graph of the first (solid line) and second (dotted line) derivatives of the conventional absorbance spectrum of the LDL-C subfraction (sigma);

Figure 8(c) is a graph of the first (solid line) and second (dotted line) derivatives of the conventional absorbance spectrum of the HDL-C subfraction (sigma);

Figure 9(a) is a graph of the first (solid line) and second (dotted line) derivatives of the Serum A test sample; and

Figure 9(b) is a graph of the first (solid line) and second (dotted line) derivatives of a Serum B test sample.

Figure 10 is a graph of the absorbance spectra for three samples wherein total cholesterol for each sample was similar but triglyceride (TG) levels varied from 30 mg/dL (Curve a), 306 md/dL (Curve b) and 630 mg/dL (Curve c).

Figures 11a, 11b and 11c are graphs respectively depicting the correlation between total cholesterol (TC), VLDL-C and LDL-C in 35 test samples as measured by the spectrophotometric active product in accordance with the present invention (denoted as "spec") and as measured by a commercially available technique (denoted "enzym").

Figures 12a and 12b are graphs depicting the correlation between HDL in 35 test samples as measured by the spectrophotometrically active product in accordance with the present invention (denoted as "spec") and as measured by a commercially available technique (denoted as "enzym").

Figures 13a, 13b and 13c are graphs depicting the correlation between TC, LDL and HDL in several hundred samples as measured by the spectrophotometrically active product in accordance with the present invention (denoted as "spec") and as measured by a commercially available technique (denoted as "enzym").

Figures 14a and 14b are graphs depicting the correlation between VLDL as measured by the spectrophotometrically active product in accordance with the present invention (denoted as "spec") and as measured by a commercially available technique (denoted as "enzym") wherein the samples had TG < 400 mg/dL (Figure 14a) and wherein TG was between 400 and 1000 mg/dL (Figure 14b).

Figures 15a and 15b are graphs depicting the inverse correlation between HDL and TG as measured by a commercially available technique (denoted as "enzym") (Figure 15a) and as measured by the spectrophotometrically active product in accordance with the present invention (denoted as "spec") (Figure 15b).

The following detailed description of the present invention is provided as an aid in the practice of the present invention.

The following discussion first provides a glossary of certain terms used herein, and then considers the inventive methods herein disclosed and concludes with a discussion of novel apparatus, which are particularly useful in performing the methods herein disclosed.

The following Glossary of Terms is provided to remove any ambiguity, which may exist as to the use of certain terms and abbreviations used herein.

The term "CD instrument" as used herein, means a Circular Dichroism Instrument. Such instruments are available commercially or may be constructed from parts, which may be commercially available. Additionally, Figure 5 is included herewith to provide a simple schematic of how a CD works. As can be seen in Figure 5, light from a light source (LS) is linearly polarized with linear polarizers (P) and then circularly polarized in opposite directions by circular polarizers (Q) and then shown through a specimen cell (S), whereupon absorbance is measured by a detector (D), the difference in absorption of the oppositely polarized light beams is measured and plotted as a function of wavelength to produce a CD spectrum, or alternatively, may be recorded at preselected wavelengths.

The term "LDL cholesterol" (abbreviated LDL-C) as used herein, means low density lipoprotein cholesterol. The term "HDL cholesterol" (abbreviated HDL-C) as used herein, means high density lipoprotein cholesterol. The term "VLDL cholesterol" (abbreviated VLDL-C) as used herein, means very low density lipoprotein cholesterol, the abbreviation "(VLDL + LDL)-C" as used herein means the combined VLDL-C and LDL-C fractions and the term "total cholesterol" (abbreviated TC) as used herein, means the sum of the cholesterol subfractions in a test sample, i.e., TC = HDL-C + LDL-C + VLDL-C. The term "cholesterol subfraction" as used herein, refers to any or all of the HDL-C, LDL-C and VLDL-C.

The term "Chugaev reagent" as used herein, means a reagent described by Cox and Spencer in <u>Can. J. Chem., 29,</u> 217 (1951) or to reagents derived from that basic reagent configuration by varying the proportions of the acetyl chloride, zinc chloride and acetic acid, or by substituting zinc acetate in acetyl chloride for zinc chloride/acetic acid.

The term "Chugaev reaction product" as used herein, means any of the reaction product(s) of cholesterol with Chugaev reagents. A "Chugaev reaction" utilized herein to form a Chugaev reaction product of the present invention, is discussed in the above-mentioned literature of Coy and Spencer and is thought to involve dehydration and opening of the B-ring of the steroid to form an optically active colored reaction product.

The term "test sample", "clinical test sample" or "serum test sample" as used herein, refers to a whole blood test sample or a whole blood test sample having the cell bodies removed therefrom by means which are well known to those skilled in the art (e.g., by centrifugal force, a filtering mechanism or the like).

The term "spectrophotometric absorption" as used herein refers to measurement of the absorption (or, conversely, transmission) of incident light by colored compounds at specific wavelengths, irrespective of the state of polarization of the light.

The term "spectrophotometric absorption detection" as used herein means detection and quantitation of analytes in a test sample by measuring their absorption of light at various wavelengths, without regard to the state of polarization of the incident or absorbed light. Absorption in this case is proportional to the number of molecules of analyte present in the test sample.

The term "fluorescence spectrophotometry" as used herein means detection and quantitation of analytes in a test sample by measuring the intensity of light emitted by the analytes at various wavelengths, following their irradiation by incident light at different wavelengths. Fluorescence is proportional to the number of molecules of analyte in the irradiated sample.

The term "first derivative spectrophotometry" as used herein describes the spectrum that is obtained by calculating the rate of change of absorbance with wavelength plotted against wavelength. The term "second derivative spectrophotometry" as used herein describes the spectrum obtained by calculating the rate of change of the first derivative with wavelength plotted against wavelength.

The term "spectrophotometrically active" or "spectrophotometric activity" as used herein refers to a property or characteristic that is detectable by spectrophotometric methods such as absorption spectrophotometry, circular dichroism, fluorescence spectrophotometry, derivative absorption spectrophotometry and the like.

## METHODS

### Direct Detection of Cholesterol Fractions Using CD Absorption, Spectrophotometric Absorption Detection, or Fluorescence or Derivative Absorption Spectrophotometric Methods

In a first embodiment, the present invention is directed to the introduction of a color reaction described in the literature, Cox and Spencer, Can. J. Chem., 29, 217 (1951), as the Chugaev reaction.

The reagents utilized in making the Chugaev reaction are for example 20% w/v $ZnCl_2$ in glacial acetic acid, and 98% acetyl chloride. These materials can be stored in separate containers and will remain usable for many weeks, even when stored at about 40°C. Moreover, the degree of their dryness does not have to be carefully controlled. The product of the Chugaev reaction with cholesterol is reddish orange in color and is thought to be a conjugated triene CD-active derivative of cholesterol. The intensity of the color is a direct measure of the cholesterol concentration. In contrast, dyes used in the known methods for cholesterol analysis are secondary products of cholesterol oxidation and are not derivatives of the cholesterol molecule itself. They are thus an indirect measure of the number of cholesterol molecules present in the test sample.

If desired, the components of the Chugaev reagent may also be stored together in a ratio over the range of about a 1:1 to 4:1 $ZnCl_2$/glacial acetic acid to 98% acetyl chloride, all of which gave satisfactory reactions with cholesterol. Reagents must be kept when stored under airtight conditions in an amber glass, Teflon® or a similar container. In this regard, an extended period of stability was observed for reactants stored together at 40°C in amber bottles for at least 4 weeks.

It was observed that when ratios of 1:1 to 4:1 of zinc reagent to acetyl chloride are utilized, voluminous precipitates can occur, which cannot always be removed in a centrifugation step that follows the incubation period. While this is not a serious problem in CD detection, because the difference in absorption of two beams is measured which effectively cancels out the contribution from light scattering, it can be serious when a single beam absorption detection method is used (e.g., absorption detection, fluorescence or derivative absorption spectrophotometry). In this respect, the invention has discovered that when the acetyl chloride is used in an amount in excess of the zinc reagent (e.g., 20%-25% w/v $ZnCl_2$ in glacial acetic acid) problems with precipitates are minimized. Most preferably the acetyl chloride is used in a high relative amount to the zinc reagent. Such preferred ratios range from 4:1 to 100:1. Alternatively, zinc acetate may be added directly to the acetyl chloride, e.g., 0.95 mg zinc acetate dihydrate in 1.0 ml acetyl chloride.

In a second embodiment, the present invention is directed to a method for forming a spectrophotometrically active product of cholesterol which comprises contacting cholesterol with an acyl compound and a perchlorate effective to form a spectrophotometrically active product of cholesterol.

Perchlorates particularly useful in this embodiment of the present invention are those which are effective to form a spectrophotometrically active product, such as a colored product, with cholesterol. Such perchlorates include but are not limited to those which contain zinc and/or barium, such as zinc perchlorate or barium perchlorate, and including hydrated forms of these; perchloric acid, which for purposes of convenience in the present specification is referred to as a perchlorate, may also be used, as may mixtures of the above. A preferred perchlorate is zinc perchlorate, such as zinc perchlorate hydrate; most especially zinc perchlorate hexahydrate.

Acyl compounds particularly useful in this embodiment of the present invention are those which, in conjunction with the above-defined perchlorates, are effective to form a spectrophotometrically active product, such as a colored product, with cholesterol. An acyl compound useful in this regard has the formula

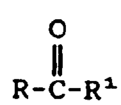

wherein $R^1$ is halogen and R is lower alkyl.

As employed herein, the lower alkyl groups contain up to 6 carbon atoms which may be in the normal or branched configuration, including methyl, ethyl, propyl, isopropyl, butyl, isobutyl, t-butyl, amyl, penty, hexyl and the like. Preferred lower alkyl groups contain 1 to 3 carbon atoms; methyl is especially preferred.

The halogens include fluorine, chlorine, bromine and iodine. The preferred halogen is chlorine.

In a preferred practice, $R^1$ is chlorine and R is methyl, resulting in acetyl chloride. Acetyl chloride is the most preferred acyl compound.

While the concentration of perchlorate and acyl compound may vary within wide ranges as determinable by those of skill in the art, it is preferred if 0.3 to 0.7 molar perchlorate is present in 90 to 100% acyl compound; more preferably 0.5 to 0.6 molar perchlorate in 95 to 99% acyl compound; most preferably 0.5 molar in 98% acyl compound. This last concentration is especially preferred when zinc perchlorate hexahydrate and acetyl chloride are employed.

In one aspect, the present invention is directed to a method for determining the amount of cholesterol present in a test sample. The cholesterol in the test sample is contacted with the aforedescribed acyl compound and perchlorate effective to form a spectrophotometrically active product with the cholesterol under conditions effective to form said product; the spectrophotometric activity of the product is then evaluated to determine the amount of cholesterol present in the sample. The spectrophotometrically active product in this regard is capable of detection by conventional techniques, such as absorption spectrophotometry, circular dichroism, fluorescence spectrophotometry or derivative absorption spectrophotometry.

In practicing the present invention, the direct, simultaneous, quantitative determination of total cholesterol and its distribution among HDL-C, LDL-C and VLDL-C subfraction in a test sample may be obtained. Thus, upon forming the spectrophotometrically active product with cholesterol in accordance with the present invention, the spectrophotometric activity of the product is measured, from which measurement is calculated the amount of HDL-C, LDL-C, VLDL-L and total cholesterol present in the test sample. Importantly, the method can be performed at ambient conditions, e.g., at about room temperature and at about atmospheric pressure, within a short period of time.

In one embodiment, it is preferred that the spectrophotometric activity of the product is measured by spectrophotometric absorption at wavelengths from between 150 to 750 nm; more preferably these measurements are made at three or more distinct wavelengths corresponding to the various subfractions among which the major portion of cholesterol is distributed. Although the wavelengths selected may vary as the choices of reagents and conditions are changed, e.g., volume of sample, volume of reagent, incubation temperature, incubation time, the actual values need not be specified since they are readily determinable by those of skill in the art. Generally, when, for example, zinc perchlorate hexahydrate and acetyl chloride are used, adequate wavelengths for the measurement of spectrophotometric absorption are at 410 nm, 456 nm and 518 nm.

From these measurements the amount of HDL-C, LDL-C and VLDL-C can be directly calculated by solving an algorithm which correlates absorption to the amounts of HDL-C, LDL-C and VLDL-C present in the samples from mathematical relations known to the art. Thus, the concentrations of HDL-C, LDL-C and VLDL-C may be calculated by solving an $n \cdot n$ matrix (where for example, $n = 3$) consisting of three linear Beer's Law equations given the sum of absorbances for each fraction as elaborated upon hereinbelow. Alternatively, the algorithm may utilize multivariate regression analysis which may comprise inter alia techniques of principal component analysis, pattern recognition analysis or partial least squares analysis, also as exemplified hereinbelow and commonly available to the art. Importantly, in this aspect of the present invention, TC is determined by the summation of the subfractions.

In practicing the present invention, the rate at which the spectrophotometrically active product is formed may be controlled, i.e., increased or decreased, by the addition of a modifier to the combination of acyl compound and perchlorate as hereinbefore defined. Modifiers useful to decrease the rate include water, glacial acetic acid, chloroform and like compounds and mixtures thereof. Generally, to decrease the rate, modifier is present at a concentration of greater than 10% v/v based upon the acyl compound, such as acetyl chloride.

Modifiers useful to increase the rate include HCl, $HClO_4$ and like compounds and mixtures thereof. Generally, to increase the rate modifier is present at a concentration of 1-2% v/v based upon the acyl compound, such as acetyl chloride.

Another aspect of this particular embodiment of the present invention is directed to a chemical reagent which comprises an acyl compound and a perchlorate effective as hereinbefore defined to form a spectrophotometrically active product with an analyte such as cholesterol.

### A. Direct Method Using CD

An advantage when using CD in the present invention is that CD allows for great specificity and selectivity in determining the amount of the different cholesterol subfractions present in the test sample, i.e., (VLDL-C + LDL-C) and HDL-C. However, a drawback is that the levels of VLDL-C and LDL-C could not be directly separated using CD.

The full CD spectrum for the orange colored optically active product from the Chugaev reaction with cholesterol is shown in Figure 1. The sample is a chloroform solution of the NBS Cholesterol Standard Reference Material (SRM9IIa). This spectrum is used as the reference standard for all subsequent serum cholesterol measurements.

In the CD absorption spectrum, the HDL-C and the (VLDL + LDL)-C fractions are associated with different spectral bands and can be measured directly from the same specimen, Figure 1, without the need for a precipitation step to determine HDL-C. In this regard, measurements at 525 nm give results for the combined (VLDL-C + LDL-C) fractions and measurements at 390 nm (or preferably the algebraic sum of the negative and positive CD absorption peaks at 390 nm and 475 nm, respectively) give results for the HDL-C fraction.

It is thought preferable to determine the algebraic sum of the CD absorption peak heights at 390 and 574 nm, when determining HDL-C levels, since this method uniformly provides a lower coefficient of variation with respect to the values obtained for HDL-C, versus the method wherein only the CD absorption measurement at 390 nm is used. The decrease in variation with the formed method results from the fact that the effects of baseline drift are lessened

when the algebraic sum of the two peaks is calculated.

In Figure 1, band assignments were made by comparing the CD spectrum for the total cholesterol, curve (a) in Figure 1, with the spectrum for the same sample after the selective precipitation of the low density lipid fractions with phosphotungstate-Mg, i.e., the shaded area in Figure 1. The 525 nm band maximum was calibrated using NBS cholesterol (SRM 911a). Calibration of the 390 nm maximum was done using secondary HDL-C calibrators supplied by Sigma Chemical Company.

As an example of carrying out one of the methods of the present invention and determining the amounts of cholesterol fractions in a test sample, there is provided Example 1:

## EXAMPLE 1

**(A) Calibration of the CD instrument:** A 50 µl aliquot of a 5 x $10^{-3}$M solution of (SRM 911a) cholesterol in AR grade chloroform is placed in a vial of 10 mL total volume. 2.00 mL of the zinc chloride reagent are added and the mixture carefully shaken. 1.00 mL of acetyl chloride is added with care, the mixture shaken, and the vial capped and incubated at 67°C for 8 minutes. The vial is removed and cooled quickly under water. Chloroform (1.00 mL) is then added to increase the solution volume in the vial. Such an addition of chloroform may be deleted if desired, if the CD analyzer will accommodate a 2.00 mL sample volume, or alternatively, an appropriate solvent substituted therefor. The solution is next transferred to a 1 cm pathlength cuvette and the CD spectrum run from 625 nm-325 nm. The spectrum is corrected on a daily basis for the cell blank and the instrument baseline by subtracting the spectrum for the reagent mixture alone.

**(b) Calibration of the CD Spectra:** the procedure in (a) is repeated for a number of solution concentrations chosen to coincide with the typical range of serum cholesterol levels in the test sample. From the resultant calibration curve the proportionality constant relating the signal size at 525 nm to the (VLDL + LDL)-C level is 1.62 millidegrees per 100 mg/dL. The calibration at 390 nm was done in the same way, but the pure cholesterol was substituted by Sigma HDL-C calibrators. The signal size to HDL-C level at 390 nm is 2.08 millidegrees per 100 mg/dL.

**(c) Cholesterol Determination in Clinical Test Samples by CD:** the procedure in (a) is repeated for 50 µL aliquots of serum. Before being transferred to the cuvette, the specimen is centrifuged at high speed for 2 minutes. The (VLDL + LDL)-C fraction is calculated from the measured signal height at 525 nm and the HDL-C fraction from the signal height at 390 nm. Their sum give the total cholesterol in the specimen. Selective precipitation of the low density fraction in order to measure the HDL-C fraction is not necessary in routine measurements. It is possible therefore, to do a cholesterol-lipid profile with a volume as little as a finger stick, and get the best precision yet obtained in the measurement of low density lipid fractions.

It should be noted that the reagents can be added in the order indicated in (a) Calibration of the Instrument. However, they can also be added simultaneously as a premixed solution or they can be added in the reverse order, e.g. add the acetyl chloride first, followed by the $ZnCl_2$ reagent. The latter mode of reagent addition had the unexpected effect of reducing the amount of precipitation in the test sample, thereby greatly reducing the scattering of incident light and thereby simplifying the subsequent measurement of absorption either by CD or by conventional spectroscopic absorption. An alternative is to use a reagent comprising zinc acetate (in lieu of zinc chloride in glacial acetic acid) and acetyl chloride.

**(d) Results of Exploratory Work:** Cholesterol determinations were made on serum samples provided by two different laboratories, which employ the commercial methods developed by Abbott Laboratories (Lab A) and DuPont (Lab B), respectively. The correlations for total cholesterol levels are excellent, Figure 2, and well within the limits imposed by the LSP.

A good case for believing that this new method is an improvement over prior methods, is to compare the correlations for the three data sets treated independently. Plots of total cholesterol versus (VLDL + LDL)-C are linear in every case, but there is a bias of almost 50 mg/dL in the intercepts on the x-axis for both conventional methods (Figures 3(b) and 3(c)) and zero correlation between the total and HDL-C data for these same data sets (Figures 4(b) and 4(c)). The Chugaev-CD data correlations by comparison, are excellent with low correlation intercepts, Figures 3(a) and 4(a), and the correlation slopes indicate that, for these sample populations, the "average" percentages for the HDL-C and (VLDL + LDL)-C fractions are 15% and 85%, respectively, which are in good agreement with the values normally accepted as typical for human serum distributions based upon ultracentrifugation data. Correlation slopes for the previously known spectrophotometric absorption methods are both one, which is not statistically possible, and which arises because the virtually constant measured value of 50 mg/dL for HDL-C is subtracted from measured TC values to obtain the results for (VLDL + LDL)-C.

**(e) Accuracy and Analysis Time:** Since there are no commercial reference standards for either LDL-C or VLDL-C, the accuracy cannot be evaluated. However, the precision and repeatability in the (VLDL + LDL)-C measurements are better than ± 2%. With this level of precision, the confidence in one's ability to correlate the changes in LDL-C in reduction therapy studies, which involved diet and/or exercise modifications, is meaningfully improved.

The approximate time for a single analysis by the Chugaev-CD method with CD detection is about 15 minutes. While this is long compared to the commercial absorption methods used only for TC measurements, results for both low and high density fractions are obtained simultaneously. Because of the stability of the color, the turn around time can be reduced considerably by incubating several samples at once. With greater incident light intensities, sample path lengths can be reduced from 1 cm and the measurements can be automated.

Utilizing Chugaev reagents in procedures such as those provided above, several National Bureau of Standards SRM total cholesterol standards were also examined. The three samples tested were listed in the NBS catalogue as (1951-1) (210.36 ± 2.46 mg/ dL total), (1951-2) (242.29 ± 1.83 mg/dL total), and (1951-3) (281.97 ± 1.83 mg/dL total). According to the NBS Certificate of Analysis, the serum was donated by the CDC. The figures in parentheses are those measured at NBS and they compare extremely well with the CDC determinations using the modified Abell-Kendall method. The figures that we obtained from the Chugaev reaction, by adding the CD absorption values for the two fractions (HDL-C and (VLD + LDL)-C) were 206 mg/dL, 241.1 mg/dL, and 286.6 mg/dL, respectively. These results clearly evidence the effectiveness of the present inventive methods in determining cholesterol levels directly and precisely.

In order to further evidence the effectiveness of the present inventive CD methods in determining levels of cholesterol subfractions in a test sample, additional experimental data are provided in Table I.

TABLE I

| Blood Fractions | | | |
|---|---|---|---|
| **Patient** | **VL + LDL(Chug)**[1] | **HDL(Chug**[2] | **HDL(enz)**[3] |
| A | 126 | 31 | [63] |
| B | 165 | 28 | [46] |
| C | 220 | 33 | --- |
| D | 237 | 34 | [55] |
| E | 199 | 29 | 32 |
| F | 188 | 39 | 36 |
| G | 249 | 36 | 43 |
| H | 199 | 34 | 25 |
| I | 144 | 28 | [53] |
| J | 216 | 46 | 52 |
| K | 190 | 38 | 35 |
| L | 211 | 41 | --- |
| M | 239 | 39 | --- |
| N | 190 | 39 | [56] |
| O | 220 | 50 | --- |
| P | 174 | 46 | 46 |
| Q | 249 | 51 | 57 |
| Q* | 242 | 48 | --- |
| R | 184 | 47 | [60] |
| S | 205 | 29 | 33 |
| T | 126 | 46 | 45 |
| U | 157 | 46 | 49 |
| U* | 163 | 41 | --- |
| V | 94 | 31 | [86] |
| W | 293 | 38 | --- |

[1] VL + LDL(Chug) - Cholesterol subfraction VLDL-C + LDL-C using Chugaev reagents and taking CD absorption measurement at 575 nm.

[2] HDL(Chug) - Cholesterol subfraction HDL-C obtained using Chugaev reagents and taking algebraic sum of Cd absorption measurements at 390 and 574 nm.

[3] HDL(enz) - subfraction HDL-C obtained using the enzymatic method designated by Lab(A) and Lab(B).

* Asterisk indicates test was performed on patient's serum using mixed Chugaev reagents stored 4 week at 40°C.

][ Brackets indicate HDL measurements which are substantially different from HDL measurements using other methods.

TABLE I (continued)

| Blood Fractions | | | |
|---|---|---|---|
| **Patient** | **VL + LDL(Chug)[1]** | **HDL(Chug[2]** | **HDL(enz)[3]** |
| X | 239 | 47 | [84] |
| Y | 207 | 57 | 55 |
| Sigma 400 | 340 | 51 | --- |
| Sigma H | 230 | 52 | --- |

[1] VL + LDL(Chug) - Cholesterol subfraction VLDL-C + LDL-C using Chugaev reagents and taking CD absorption measurement at 575 nm.

[2] HDL(Chug) - Cholesterol subfraction HDL-C obtained using Chugaev reagents and taking algebraic sum of Cd absorption measurements at 390 and 574 nm.

[3] HDL(enz) - subfraction HDL-C obtained using the enzymatic method designated by Lab(A) and Lab(B).

Of the experimental results shown in Table I, it is noted that 12 out of 20 values for each of the HDL-C(Chug) and HDL-C(enz) methods agree to within 10 mg/dL. Such results clearly help to evidence the accuracy of the present methods.

**B. Direct Detection Using Spectrophotometric Absorption Detection**

The visible absorption spectrum for the colored product of the Chugaev reaction with cholesterol or serum cholesterol shows a strong maximum around 518 nm, a minimum around 460 nm, with shoulders at wavelengths between 460 nm and 365 nm showing a fairly weak absorption maximum (Figure 6). Additionally, the Chugaev reagent itself absorbs in the visible range and has a weak maximum in the 350-370 nm regions.

In order to ascertain the effectiveness of the present inventive spectrophotometric absorption detection methods, samples of separated HDL-C, VLDL-C, and LDL-C lipoprotein fractions were obtained from Sigma Chemical Company (Sigma; fractions separated by ultrafiltration) and from Oklahoma Medical Research Foundation (OMRF; subfractions separated by ultracentrifugation). The three subfractions were reacted separately with Chugaev reagents to give colored products which possess different absorption spectra in the visible range. Spectral correspondences between the fractions from the two different sources were excellent for the HDL-C and LDL-C samples. Correspondence for the VLDL-C subfractions were also good. The spectral differences between each of the subfractions is sufficient to enable the three cholesterol subfractions to be qualitatively determined simultaneously in a single experiment without resorting to a selective precipitation step. For purposes of this example, absorption measurements are taken at 518, 450 and 420 nm. The serum spectrum of any test sample is an aggregate of the weighted contribution from each subfraction.

The ability to calculate the amount of each cholesterol subfraction present in a test sample, is due to the inventors' initial postulation that all three subfractions absorb at every wavelength analyzed, so that the general equation for total absorbance $A_T$ of a serum test sample is given by the equation:

$$E_{HDL}[HDL] + E_{VLDL}[VLDL] + E_{LDL}[LDL] = A_T$$

In the above equation, the E coefficients denote the absorbances for each of the subscripted fractions normalized in appropriate units of absorbance/(mg/dL), and the concentration terms [ ] are in mg/dL. Utilizing the above equation, and making the further assumption that each subfraction has the same or a substantially similar absorption coefficient at 518 nm but, as exemplified by the different spectra, have different absorption coefficients at the other wavelengths (in this case 420 and 450 nm), it is possible to calculate the amount of each subfraction present in a test sample by taking an $A_T$ measurement at each of the three discrete wavelengths in the spectrum and solving the resulting $3 \cdot 3$ matrix equation. In order to do this, the individual values for the subscripted E coefficients have to be determined for the three wavelengths selected. Relative values for the E coefficients at different wavelengths were easily obtained within the spectrum of any one of the subfractions. Relating these to the values of the equivalent wavelengths for the other fractions was more difficult, but was achieved and is disclosed herein. In this respect, spectral analysis of about 90 serum samples showed that a direct linear correlation existed between the $A_T$ values measured at 518 nm and the value for total cholesterol (TC) measured in a completely independent study that utilized a conventional method (data from Roche Biomedical). Based on this linear correlation, the inventor presupposed that a normalized value expressed as $A_T$/TC should be constant from sample to sample.

In order to correlate E values between the spectra for the different subfractions, the inventor postulated that the $A_T$/TC (or E) values at 518 nm are the same for all three subfractions, as noted above, and that the remaining six

E coefficients for the three subfractions can be calculated for the remaining two wavelengths using simple proportions (e.g., $E_{LDL(420)} = A_{T(420)}/A_{T(518)} \times E_{LDL(518)}$.

The amount of the above three lipofractions calculated from 60 serum samples utilizing the above technique and 3 · 3 matrixes provided results in excellent agreement with numbers obtained for the same test samples utilizing a conventional reaction procedure.

The specific test procedure utilized with the 60 samples that gave excellent agreement was as follows. After reagents were added the mixtures were allowed to incubate for 8 minutes at 67°C, thereafter cooled in a waterbath, centrifuged, transferred to a one cm cuvette, and a conventional absorption spectrum run from 700 nm-400 nm. Absorption measurements were taken at 518, 450 and 420 nm. after appropriate corrections for the cell blank and the instrument base line were made. It is not necessary to run the entire spectrum, since absorbance measurements are only needed at the prescribed wavelengths. The nine E values for the wavelengths 518, 450 and 420 nm, respectively, obtained under the above described particular experimental conditions in units of absorbance, dL/gram were as follows: 3.05, 1.97 and 2.52 for HDL-C; 3.05, 1.35 and 2.41 for VLDL-C; and 3.05, 1.31 and 1.34 for LDL-C.

With Chugaev reagents problems may arise in the use of absorption spectrophotometry which do not exist when CD methods are used. Specifically, whenever an absorption detection spectrophotometric method is utilized with Chugaev reagents precipitation may create problems. In order to fully minimize such problems, the Chugaev reagent should be modified such that either the ratio of acetyl chloride to zinc reagent is from 100:1 to 4:1 or zinc acetate is substituted for zinc chloride/acetic acid. The final zinc concentration should be between 0.03 and 0.22 molar. This is a composition much different from the reagent described in the literature.

It is noted that excellent absorption spectra have been obtained for volumes of serum as little as 2 µl. Likewise, excellent spectra were obtained for various acetyl chloride to serum ratios over the general broad range of 100:1 to 20:1 at constant zinc concentrations, and for acetyl chloride to zinc reagent ratios from 100:1 to 4:1 at constant serum amounts. Measurements have also been made with test samples, wherein the total reaction volume was as little as 0.15 mL and in cuvettes having a pathlength as short as 1 mm. Moreover, incubation times as little as two minutes have been achieved with the smaller total volumes, and it is fully envisioned that under conditions where serum concentrations are relatively high, that lower incubation temperature may be utilized. Furthermore, with appropriate miniaturization, centrifugation may be eliminated.

In addition to the Chugaev reagent system described above, it is noted that ACS reagent grade zinc acetate dihydrate readily dissolves in acetyl chloride to a concentration that is similar to the final zinc ion concentration when added as the chloride in glacial acetic acid. Zinc acetate in acetyl chloride therefore can work, if desired, as a single reagent system. For example, if one mL of such a reagent system is added to 10 µL of a test serum, there is obtained a reddish-orange product after the usual incubation conditions. The maxima and minima in the spectra are at the same wavelengths but the ratios of the heights of these bands are different from those seen with the zinc chloride reagents with the unexpected finding of a greater difference between the absorbances at 420 and 450 nm. Consequently, new E coefficients would need to be calculated for a 3 · 3 matrix if the zinc acetate were used rather than zinc chloride in acetic acid. The greater difference at those wavelengths means better precision in the values obtained for the subfractions. Indeed, the coefficients are highly dependent on the composition of the reagent and must be recalculated if the amounts of any of the reagent components are changed. However, such a calculation is in line with those earlier described, and clearly within the skill of those of ordinary skill in the art, based on the present disclosure.

A broad range of alternative reaction conditions to those reaction conditions discussed above, will produce reddish-orange colored products which have spectra that are similar, but not always equivalent to the absorbance spectrum of the species produced under the exact conditions utilized herein (as described above). Even so, when those skilled in the art utilize such alternative reaction conditions in combination with the 3 · 3 matrix strategy provided herein, and calculate the amounts of each subfraction present, they are practicing the inventor's presently disclosed methods. This is true, even though the nine E coefficients utilized may have to be revised after a recalibration of the spectra for standards of each of the subfractions (e.g., Sigma or OMRF provided subfractions) based on the exact reaction conditions employed. As such, it is envisioned that the present spectrophotometric methods clearly cover all such possible reagent mixtures and reagent ratios, so long as a colored reaction product is formed with the cholesterol subfractions, and the amounts of each subfraction are then determined in a manner as described above.

The above-described spectrophotometric absorption methods offer an opportunity for simultaneous, on-line detection of cholesterol and cholesterol subfractions in clinical samples. The use of spectrophotometric absorption methods in accordance with the present invention also permits much greater sensitivity than the CD methods herein disclosed allow for, since only a very small portion of the incident light can be used for CD signal generation. As such, the spectrophotometric absorption methods herein disclosed also permit the use of smaller volumes of sample, thereby reducing possible interferences caused by other materials and the total amount of precipitates formed by the reaction. Conversely, however, these reactions are more susceptible than CD to interferences from pigments released by hemolysis of the blood samples. Finally, it is important to note that, as with the CD studies mentioned above, addition of the acetyl chloride to the sample first, followed by addition to the $ZnCl_2$/acetic acid reagents reduces even further the

interferences caused by precipitation in a clinical sample. Indeed it is possible to carry out spectrophotometric absorbance reactions in the present inventive methods using whole blood samples.

In the following Table II, there is provided comparative data obtained with test samples using both the spectrophotometric absorption/Chugaev method disclosed herein and an enzymatic method for the cholesterol subfractions shown. As may be seen upon review of Table II, excellent results were obtained using the Chugaev reagents/spectrophotometric absorption method herein described (as verified by comparing with results obtained on the same test samples using the enzymatic method).

TABLE II:

| Subfractions from Enzymatic and Chugaev Methods | | | | | |
|---|---|---|---|---|---|
| **Test Subject** | **Test** | **VLDL-C** | **LDL-C** | **HDL-C** | **TC** |
| 1 | enzymatic | 32 | 155 | 28 | 216 |
| | Chugaev | 29 | 155 | 35 | 222 |
| 2 | enzymatic | 46 | 178 | 37 | 262 |
| | Chugaev | 50 | 173 | 42 | 264 |
| 3 | enzymatic | 37 | 110 | 36 | 183 |
| | Chugaev | 48 | 103 | 31 | 182 |
| 4 | enzymatic | 34 | 133 | 46 | 214 |
| | Chugaev | 38 | 139 | 36 | 213 |
| 5 | enzymatic | 42 | 155 | 45 | 242 |
| | Chugaev | 44 | 161 | 41 | 246 |
| 6 | enzymatic | 62 | 113 | 26 | 202 |
| | Chugaev | 65 | 101 | 35 | 203 |

Based on the above considerations, there is provided herein a novel spectrophotometric absorption detection method, wherein cholesterol subfractions in clinical samples are reacted with either Chugaev reagents or the acyl compound and perchlorate reagent system as defined hereinabove, so that a direct measurement of the cholesterol subfractions can be made. The measurements can be made either as a full spectrum over the range of about 150-700 nm or at 3 selected wavelengths, in this case about 420 nm. 450 nm and 518 nm.

The major procedural difference between the absorption and the CD method relates to the standards used. While cholesterol itself can be used as a standard for the CD reactions, clinical standards for TC and cholesterol subfractions obtained from the CDC, CAP or a commercial source must be used to calibrate the absorption spectrometer.

Further to the above disclosed spectrophotometric methods, given the availability of "pure" samples of VLDL-C, LDL-C and HDL-C, a mathematical algorithm can be prepared, if so desired, which enables one to add the individual subfractions' spectrophotometric absorption spectra in a weighted fashion for each subfraction. In such a manner the total absorption spectrum for the test sample is obtained. Utilizing such a method would be analogous to measuring the spectrophotometric absorption of a colored reaction product at an infinite number of points, instead of just at three or more distinct points as described above.

As an example of practicing the material of the present invention whereby a spectrophotometrically active product of cholesterol in a test sample is formed by contact with an acyl compound and a perchlorate as hereinbefore defined, Example 2 is provided.

## EXAMPLE 2

**Experimental Conditions:**

The reagent consisted of an 0.5 M solution of zinc perchlorate six hydrate [$Zn(ClO_4)_2 \cdot 6H_2O$)] in 98% acetyl chloride. After mixing, the solution was centrifuged to remove suspended materials, most probably undissolved ZnO, which is reported by the manufacturer to be a possible impurity. The reagent was stable when stored in a tightly sealed

amber-glass container.

A 20 µL aliquot of serum was placed in a glass or polypropylene vial and 2 mL of reagent was carefully added and the mixture was shaken thoroughly. Transfer proteins were precipitated on the addition of the reagent and were quickly and easily removed either by centrifugation or filtration. The supernate was transferred to a sealed, 1 cm pathlength, spectrophotometric cuvette and allowed to stand at room temperature for 15 minutes at which time the absorption spectrum was measured from 750-380 nm. The instrument used was a diode array spectrophotometer, but the necessary data for the lipid profile analysis was obtained using only four wavelengths, one of which (700 nm) was used for the sample baseline correction. The other wavelengths were 410 nm. 456 nm and 518 nm.

The values for total cholesterol in a sample was determined from the absorbance measured at the major maximum, which under the above experimental conditions occurred at 518 nm. The constant that allowed total cholesterol to be calculated was obtained from the slope of the linear correlation between measured absorbance $A_T$ (518) and the total cholesterol measured by the commercially available enzymatic method. The mathematical procedure to calculate the concentration of the three major fractions, VLDL-C, LDL-C and HDL-C was the same as hereinbefore described, i.e., by solving the 3 x 3 matrix that consists of three linear Beer's Law equations given by the sum of the absorbance for each fraction:

$$A_{T(i)} = A_{VLDL\text{-}C(i)} + A_{LDL\text{-}C(i)} + A_{HDL\text{-}C(i)}$$

wherein i = 1-3.

Each A term for a fraction consisted of a product of the concentration of the fraction times a coefficient E : e. g., $A_{LDL\text{-}C(i)} = E_{LDL\text{-}C(i)} \cdot$ [LDL-C]. E values are usually defined as molar absorbances but for this application the units were converted to mg/dL which is standard for reporting lipid data. The solution required that the nine E coefficients (one for each fraction at three wavelengths) be determined. This could not be done exactly because there are no known samples of reference standards available for the lipid fractions. Therefore, they were arrived at empirically. This was done by comparing lipid panels for a large pool of serum samples that had been measured by the commercially available enzymatic method and by the method of the present invention. The coefficients were systematically adjusted as the size of the pool was enlarged to give the best statistical fit among data for all the fractions.

The results for the lipid profile analysis is presented below in Table III. Enzymatic refers to the independent results measured by Roche Biomedical, Kansas City; spectrophotometric refers to the results from the Chugaev reaction described herein; and kinetic refers to the results from the use of zinc perchlorate hexahydrate and acetyl chloride.

TABLE III

| Comparisons of Lipid Panels by Three Methods | | | | | |
|---|---|---|---|---|---|
| | A | B | C | D | E |
| 1 | Method | | | | |
| 2 | | Total | VLDL | LDL | HDL |
| 3 | Sample 1 | | | | |
| 4 | enzymatic | 160 | 20 | 100 | 40 |
| 5 | spectrophotometric | 170 | 25 | 100 | 45 |
| 6 | kinetic | 150 | 12 | 90 | 48 |
| 7 | | | | | |
| 8 | Sample 2 | | | | |
| 9 | enzymatic | 179 | 13 | 124 | 41 |
| 10 | spectrophotometric | 181 | 13 | 121 | 47 |
| 11 | kinetic | 165 | 5 | 113 | 47 |
| 12 | | | | | |
| 13 | Sample 3 | | | | |
| 14 | enzymatic | 215 | 39 | 141 | 35 |
| 15 | epectrophotometric | 229 | 36 | 131 | 62 |

TABLE III (continued)

| Comparisons of Lipid Panels by Three Methods | | | | | |
|---|---|---|---|---|---|
| | A | B | C | D | E |
| 16 | kinetic | 208 | 23 | 126 | 58 |
| 17 | | | | | |
| 18 | Sample 4 | | | | |
| 19 | enzymatic | 204 | 26 | 141 | 36 |
| 20 | spectrophotometric | 213 | 24 | 132 | 57 |
| 21 | kinetic | 195 | 20 | 125 | 49 |
| 22 | | | | | |
| 23 | Sample 5 | | | | |
| 24 | enzymatic | 195 | 68 | 98 | 28 |
| 25 | spectrophotometric | 217 | 52 | 106 | 59 |
| 26 | kinetic | 202 | 46 | 104 | 52 |
| 27 | | | | | |
| 28 | Sample 6 | | | | |
| 29 | enzymatic | 196 | 26 | 129 | 41 |
| 30 | spectrophotometric | 200 | 20 | 131 | 49 |
| 31 | kinetic | 198 | 23 | 126 | 49 |
| 32 | | | | | |
| 33 | Sample 7 | | | | |
| 34 | enzymatic | 233 | 39 | 151 | 42 |
| 35 | spectrophotometric | 243 | 28 | 153 | 62 |
| 36 | kinetic | 229 | 28 | 148 | 54 |
| 37 | | | | | |
| 38 | Sample 8 | | | | |
| 39 | enzymatic | 132 | 36 | 65 | 31 |
| 40 | spectrophotometric | 135 | 36 | 60 | 39 |
| 41 | kinetic | 119 | 27 | 61 | 30 |
| 42 | | | | | |
| 43 | | | | | |
| 44 | Sample 9 | | | | |
| 45 | enzymatic | 232 | 19 | 160 | 50 |
| 46 | spectrophotometric | 231 | 15 | 156 | 60 |
| 47 | kinetic | 231 | 18 | 164 | 49 |
| 48 | | | | | |
| 49 | Sample 10 | | | | |
| 50 | enzymatic | 168 | 14 | 109 | 44 |
| 51 | spectrophotometric | 177 | 21 | 108 | 48 |
| 52 | kinetic | 153 | 20 | 100 | 34 |

TABLE III (continued)

| Comparisons of Lipid Panels by Three Methods | | | | | |
|---|---|---|---|---|---|
| | A | B | C | D | E |
| 53 | | | | | |
| 54 | Sample 11 | | | | |
| 55 | enzymatic | 170 | 46 | 95 | 28 |
| 56 | spectrophotometric | 184 | 56 | 85 | 43 |
| 57 | kinetic | 178 | 32 | 100 | 45 |
| 58 | | | | | |
| 59 | Sample 12 | | | | |
| 60 | enzymatic | 186 | 10 | 113 | 62 |
| 61 | spectrophotometric | 193 | 21 | 119 | 53 |
| 62 | kinetic | 170 | 10 | 108 | 50 |
| 63 | | | | | |
| 64 | Sample 13 | | | | |
| 65 | enzymatic | 201 | 30 | 111 | 59 |
| 66 | spectrophotometric | 215 | 49 | 104 | 62 |
| 67 | kinetic | 208 | 24 | 128 | 58 |
| 68 | | | | | |
| 69 | Sample 14 | | | | |
| 70 | enzymatic | 180 | 26 | 113 | 40 |
| 71 | spectrophotometric | 186 | 36 | 97 | 53 |
| 72 | kinetic | 186 | 24 | 116 | 46 |
| 73 | | | | | |
| 74 | | | | | |
| 75 | Sample 15 | | | | |
| 76 | enzymatic | 186 | 17 | 127 | 41 |
| 77 | spectrophotometric | 194 | 28 | 111 | 55 |
| 78 | kinetic | 182 | 21 | 120 | 44 |
| 79 | | | | | |
| 80 | Sample 16 | | | | |
| 81 | enzymatic | 175 | 15 | 106 | 54 |
| 82 | spectrophotometric | 186 | 26 | 111 | 49 |
| 83 | kinetic | 180 | 24 | 119 | 37 |
| 84 | | | | | |
| 85 | | | | | |
| 86 | | | | | |
| 87 | Sample 17 | | | | |
| 88 | enzymatic | 173 | 9 | 113 | 50 |
| 89 | spectrophotometric | 172 | 22 | 102 | 48 |

TABLE III (continued)

| Comparisons of Lipid Panels by Three Methods | | | | | |
|---|---|---|---|---|---|
| | A | B | C | D | E |
| 90 | kinetic | 176 | 18 | 118 | 39 |

**EXAMPLE 3**

In this example, zinc perchlorate hexahydrate and acetyl chloride were contacted with cholesterol in a test sample following substantially the procedure described in Example 2; however, the calculation of cholesterol in this example was by way of multivariate regression analysis instead of a 3 x 3 matrix used in Example 2.

Incubation was performed for 15 minutes at ambient temperature. Protein that precipitated on addition of a 2 mL aliquot of zinc perchlorate hexahydrate/acetylchloride reagent to 20 µL of serum was removed by centrifugation or by filtration. The batching of samples reduced the time per test to approximately 5 minutes.

**Absorbance Measurements:** Full spectral (350-750 nm) absorbance data for a sample with a pathlength of 1 cm were collected on a Hewlett Packard 8452A diode array spectrophotometer; accumulation time was 5 seconds. With the speed and convenience of diode array detection technology, the wavelength range was wider than that used for the method employing Chugaev reagents.

**Computational:** Since pure forms of cholesterol lipid fractions are not available, neither are the spectra for the individual products of the color reaction. Therefore a mathematical model was developed to resolve the whole spectrum into the contributions from the parts.

**(a) 3 x 3 Matrix Solution:** In Example 2, absorbances, $A_{(i)}$, were measured at three principal wavelengths and the lipid profiles were calculated by solving a set of three simultaneous equations of the form:

$$A_{(i)} = E_{VLDL(i)}[VLDL]d + E_{LDL(i)}[LDL]d + E_{HDL(i)}[HDL]d$$

where d was the sample pathlength. The nine E coefficients were evaluated in an empirical manner.

**(b) Multivariate Regression Analysis (MVRA):** Lipid profile and TC results in this example were determined using MVRA techniques to interpret the full absorbance spectrum, which eliminated simplifying assumptions and investigator bias. The MVRA algorithms that were applied were Partial Least Squares 2 (PLS2) and Principal Component Analysis (PCA) using software for spectroscopic analysis available in the commercial package **UNSCRAMBLER II** (CAMO A/S, Trondheim, Norway). Spectral resolution in the spectrophotometer was 2 nm so a full spectrum consisted of 200 data points, which represented an enormous increase in the number of degrees of freedom compared to the simpler analysis of Example 2.

**Training Set:** An analytical model was prepared by compiling a training set that consisted of 35 serum samples for which the lipid profiles had been measured by commercially available enzymatic techniques; VLDL-C here was taken to be 0.2 x TG and numbers of LDL-C were calculated using the Friedewald formula. Ranges in values were as wide as could be accessed, namely; 8-80 mg/dL for VLDL-C; 85-222 mg/dL for LDL-C; and 20-80 mg/dL for HDL-C. Using both the PLS2 and PCA algorithms, the optimum fit to the spectral data for these 35 samples was obtained using three factors. The percent residual variance that was observed was about 25% with only the first factor and less than 0.05% for all three. No corrections were made for noise or background and no weighting corrections were introduced.

As a test to determine if full spectral analysis was really necessary, the MVRA subroutines were used to identify the optimum wavelengths, i.e., those that are most sensitive to variations in the amounts of each fraction. Alternative models were prepared using reduced data sets limited to 100, 30, 14, 6 and 4 wavelengths respectively. Little difference was seen in the percent residual variance through the model with 6 points, and 4 could be used with little loss in the quality of the fit. For purposes of the present example, absorbances were measured at six wavelengths.

**Sample Predictions:** The above-described analytical model was used to predict lipid profiles for several hundred samples which were about equally split between regular TG and high TG levels. The major source of full lipid profile data for comparisons between methods was Roche Biomedical, Kansas City, which utilized commercially available enzymatic techniques. The major source for HTG samples was the Stillwater Medical Center. For the latter, only TC and TG levels were reported.

The formation of the spectrophotometrically active product was performed with the HTG samples in precisely the same way and it was assumed that the same model could be extrapolated to include them. Four of the thirty five in the original training set were HTG samples. For these four, TG levels were slightly above 400 mg/dL; HDL values

were measured, and lipid profiles calculated.

In this treatment, values for the fractions were determined directly and TC was calculated from their sum. Thus, unlike the enzymatic methods, TC was not measured experimentally.

**Absorbance Spectra:** A comparison of spectra for the colored products from one regular and two HTG serum samples is shown in Figure 10. All three had a TC of about 186 mg/dL, as measured enzymatically. The critical part of the spectrum for discrimination among the lipid fractions was in the range of about 360 to about 480 nm.

**Training Set:** As seen in Figure 11, very good linear correlations for VLDL-C, LDL-C and TC between methods was obtained for the training set. As seen in Figure 12, the linear correlation for HDL-C was not nearly as good, but it showed an improvement over the Chugaev-reagent method. The result from a paired t-test suggested that there was a correlation, and further evidence to support such a correlation was given by the shape of the bivariate ellipse which, as shown in Figure 12, was drawn with a confidence region of 65%, which meant that for a normal distribution in X and Y, 65% would lie within the ellipse.

**Predictions:**

**Regular Samples:** As shown in Figure 13(a), the between-methods linear correlation for TC was excellent. The range was from about 58 to about 500 mg/dL and the figure included data for HTG as well as regular samples. This was significant because TC is measured in one method and calculated in the other, which validated the MVRA models used for the spectral interpretation.

As shown in Figure 13(b), the between-methods linear correlations were also very strong for the LDL-C fraction, which suggested that the measurement of LDL-C by the direct method of the present invention was reliable. As shown in Figure 13(c), the correlation of HDL-C was only slightly worse than it was for the training set. The VLDL-C correlation for samples with TG less than about 400 mg/dL was good, as shown in Figure 14(a).

**HTG Samples:** Absorbances in the range of about 360 to about 430 nm increased dramatically with increasing TG. It was significant that the increase in the maximum absorbance at 360 nm was non-linear with the amount of TG, as shown in Figure 10. A linear dependence would only be expected if the band could be assigned entirely to VLDL-C absorption and the approximation VLDL-C = 0.2 TG were true for HTG samples, which it was not. Meaningful results for lipid profiles were obtained for HTG samples where the TG level was as high as 2000 mg/dL. Between 400 and 1000 mg/dL, the VLDL-C/TG ratio was seen to decrease from 0.2 to 0.12 which is manifested in the curvature observed in the plots of VLDL-C (spec) vs. TG, as shown in Figure 14(b). This was consistent with the fact that the Friedewald equation, with VLDL-C = 0.2 x TG, fails at high TG levels.

An inverse correlation between HDL-C and TG has sometimes been alluded to. As long as the routine measurement of VLDL-C was limited to 0.2 x TG and TG values were less than TG = 400 mg/dL, the correlation had not been obvious. As shown in Figure 15, adding VLDL-C data for HTG samples emphasized the relationship.

**C. Direct Detection Using Fluorescence and Derivative Absorption Spectrophotometric Methods**

The products of the reaction of cholesterol with the Chugaev reagents are fluorescent. Moreover, fluorescence spectra for the three lipoprotein subfractions VLDL-C, LDL-C and HDL-C are different from each other and from the spectrum for a serum sample (see Figures 7(a), 7(b) and 7(c)). The mathematical analysis of fluorescence data, wherein one calculates the amounts of each of the subfractions present in a serum test sample is entirely equivalent to that described above for the conventional absorbance detection spectrophotometry. All that is required to initiate the calculation are the nine fluorescence coefficients for whatever three wavelengths are selected in the fluorescence spectrum for serum. In this respect, wavelengths different from those utilized in conventional absorption spectrophotometry are needed, since the maximum and minimum wavelengths for fluorescence occur at longer wavelengths.

Similarly, derivative absorption spectrophotometry may also be utilized to calculate the amount of a cholesterol subfraction present in the test serum sample. For example, first and second derivatives of absorbance spectra can be utilized for analytical measurements. Copies of derivative absorbance spectra for each of the three lipoprotein subfractions are shown in Figure 8. Figure 8(a) shows the first and second derivative for VLDL-C; Figure 8(b) shows the first and second derivative for LDL-C; and Figure 8(c) shows the first and second derivative for HDL-C. In each of Figures 8(a)-8(c), the solid line denotes a first derivative to the absorbance spectrum and the dotted line denotes the second derivative of the absorbance spectra. Each of the subfractions utilized to obtain the graphs 8(a)-8(c) were obtained from Sigma Chemical Company. When utilizing derivative absorption spectrophotometry, subtle differences exist between the spectra for each of the fractions. Again, the mathematical analysis is completely analogous to that discussed above for absorbance detection and fluorescence spectrophotometries. However, three new wavelengths would need to be chosen. Signal intensities at the band maxima are much better separated than with other methods and precision may therefore be increased. In two measurements on serum samples (see Figures 9(a) and 9(b)) it was determined that the peak to peak heights for the two major bands were directly proportional to TC. Data collection

utilizing derivative spectrophotometry requires the use of a full spectrum analysis.

**INVENTIVE APPARATUS**

Upon review of the above methods section, it can be easily ascertained that the present inventive methods have many advantageous attributes when compared with presently known methods for determining cholesterol levels in test samples. However, the present invention also encompasses novel instruments, which allow those skilled in the art to practice the present inventive methods. Such inventive instruments are outlined above (see Section entitled "Summary of the Invention").

A spectrophotometric instrument encompassed hereby should be equipped with 1 or more spectrophotometric absorption detectors capable of measuring the absorption of the colored products of the Chugaev reagent over a range of from about 360-700 nm, or at discrete points therein such as about 518 nm, 450 nm and 420 nm. If automated, it should also have the capability of adding the reagents to separate sample containers for analysis or to sequentially add the components of the reagents to minimize problems due to precipitation of proteins. Finally, any such absorption spectrophotometer, manual or automatic, should preferably have the means to determine the levels of each subfraction present in a serum test sample by a calculation or computation from the absorption values obtained. Specifically, the instrument should have the ability to compute the results of the 3 · 3 matrix, with nine pre-programmed constants, to establish the levels of VLDL-C, LDL-C and HDL-C present, and to use these values to compute the TC present in the sample, or alternatively, should be equipped with the ability to employ multivariate regression analysis to establish these levels.

It should be noted that the 3 · 3 matrix represents the minimum possible to measure the three subfractions. It is possible that finer analysis of the spectrum produced by the reagent will indicate that constants at other specific wavelengths will provide useful information, e.g., about specific molecular entities within the various subfractions. In that case, the instrument should be construed to analyze matrixes larger than 3 · 3.

**EXAMPLE 4**

This example describes the use of modifier to control the rate of reaction using acyl compound and perchlorate.

**Modification of Reagent to Manage Reaction Rates:**

The rate of the color reaction and the reagent using acetyl chloride and zinc perchlorate hexahydrate can be increased or decreased by using a modifier in the following manner:

(a) a rate decrease by a factor of two or more was observed when either water, or glacial acetic acid, or chloroform at a level greater than 10% v/v was added to acetyl chloride. Water must be added with great care because of the heat of mixing. At water levels greater than 20% a blue colored solution was produced due to reaction with protein. At 50% chloroform the reagents were immiscible. The rate was controlled by selecting the appropriate mole fraction for the added solvent. Retardation of the reaction at 25°C by the addition of any of these solvents can be used to advantage if a temperature of 37°C is preferred for the reaction.

(b) a rate increase was observed when strong acid (HCl or $HClO_4$) was added in the amount of 1.2% v/v. Again great care must be used when adding the aqueous acids. The spectrum after 10 minutes was the same as that for the unmodified reagent after 15 minutes, but the spectrum for the acid mixture changes considerably over the next 5 five minutes so more careful control of the condition would be necessary.

Zinc perchlorate hexahydrate dissolved easily in acetyl chloride and the reagent has a relatively long shelf life in a sealed container under ambient conditions. The shelf life was extended when the reagent was stored in the refrigerator. With some commercial products a slight amount of insoluble material is left. This was readily removed by slow speed centrifugation and the data for this reagent compared exactly with data where the zinc perchlorate completely dissolved. For measurements at a temperature of 25°C the procedure called for the thorough mixing of 2 mL of the modified single reagent with a 20-50 µL aliquot of serum, centrifuging (or filtering) the mixture to remove precipitated proteins, and measurement. The visible spectrum of the product changed with time and the reaction was approximately 95-98% complete after 15 minutes at which time the absorbance spectrum was measured from 700-400 nm against a reagent blank. The spectrum for the colored product after 15 minutes is analogous to that observed for the zinc chloride (modified Chugaev) reagent with a slight blue shift in the wavelengths of the minimum and second maximum.

Calculations of TC and its distribution among the three major lipoproteins were done in exactly the same way

as before, e.g., a 3 x 3 matrix of linear Beer's Law equations are set up for three distinct wavelengths, at 518, 456 and 410 nm. Reagent blank and instrument baseline were measured at 700 nm. Absorbance coefficients were determined empirically as before, by first assuming that all three fractions had the same molar absorbance at 518 nm and subsequently ratio-ing respective absorptions at 518 nm. For VLDL-C the current adjusted coefficients are: 2.70, 2.90; for LDL-C they are: 2.70, 1.15 and 1.20; and for HDL-C they are: 2.70, 1.25 and 2.30.

## Claims

1. A method for forming a spectrophotometrically active product of cholesterol which comprises contacting cholesterol with an acyl compound having the formula:

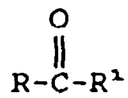

wherein $R^1$ is halogen, R is lower alkyl; and a perchlorate effective to form a spectrophotometrically active product of said cholesterol, said perchlorate selected from the group consisting of zinc perchlorate, barium perchlorate and perchloric acid.

2. The method of Claim 1 wherein said cholesterol is distributed among HDL-C, LDL-C and VLDL-C subfractions.

3. The method of Claim 1 wherein said perchlorate is zinc perchlorate hexahydrate.

4. The method of Claim 1 wherein $R^1$ is chlorine and R is methyl.

5. The method of Claim 1 wherein said perchlorate is present in a concentration of 0.3-0.7 molar in 90-100% acyl compounds.

6. The method of Claim 5 wherein said perchlorate is present in a concentration of 0.4-0.6 molar in a concentration of 95-99% acyl compound.

7. The method of Claim 6 wherein said perchlorate is present in a concentration of 0.5 molar in 98% acyl compound.

8. The method of Claim 7 wherein said perchlorate is zinc perchlorate hexahydrate and said acyl compound is acetyl chloride.

9. The method of Claim 7 wherein said perchlorate is perchloric acid and said acyl compound is acetyl chloride.

10. The method of Claim 1 wherein said spectrophotometrically active product is a colored product.

11. A method for determining the amount of cholesterol present in a test sample which comprises contacting a test sampel in which cholesterol is present with an acyl compound having the formula:

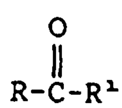

wherein $R^1$ is halogen, R is lower alkyl, and a perchlorate effective to form a spectrophotometrically active product with said cholesterol, said perchlorate selected from the group consisting of zinc perchlorate, barium perchlorate and perchloric acid, and evaluating said spectrophotometric activity to determine the amoutn of said cholesterol present in said sample.

12. The method of Claim 11 wherein said cholesterol is distributed among HDL-C, LDL-C and VLDL-C subfractions.

13. The method of Claim 12 wherein said perchlorate is perchloric acid and R is lower alkyl.

**14.** The method of Claim 13 wherein R is methyl.

**15.** The method of Claim 12 wherein said zinc perchlorate is a zinc perchlorate hydrate and R is lower alkyl.

**16.** The method of Claim 15 wherein said perchlorate is zinc perchlorate hexahydrate and said acyl compound is acetyl chloride.

**17.** The method of Claim 11 wherein said perchlorate is present in a concentration of 0.3-0.7 molar in 90-100% acyl compound.

**18.** The method of Claim 17 wherein said perchlorate is present in a concentration of 0.4-0.6 molar in 95-99% acyl compound.

**19.** The method of Claim 18 wherein said perchlorate is present in a concentration of 0.5 molar in 98% acyl compound.

**20.** The method of Claim 19 wherein said perchlorate is zinc perchlorate hexahydrate and said acyl compound is acetyl chloride.

**21.** The method of Claim 19 wherein said perchlorate is perchloric acid and said acyl compound is acetyl chloride.

**22.** The method of Claim 11 wherein said spectrophotometrically active product is capable of detection by circular dichroism, absorption spectrophotometry, fluorescence spectrophotometry or derivative absorption spectrophotometry.

**23.** The method of Claim 11 wherein said spectrophotometric activity is evaluated by measuring the absorption spectrum of said product at wavelengths from between 150 to 750 nm.

**24.** A method for direct, simultaneous, quantitative determination of total cholesterol and its distribution among HDL-C, LDL-C and VLDL-C subfractions in a test sample which comprises contacting a test sample containing cholesterol distributed among HDL-C, LDL-C and VLDL-C subfractions with an acyl compound having the formula:

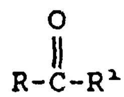

wherein $R^1$ is halogen, R is lower alkyl; and a perchlorate effective to form a spectrophotometrically active product with said cholesterol, said perchlorate selected from the group consisting of zinc perchlorate, barium perchlorate and perchloric acid; measuring the spectrophotometric absorption of said product; and calculating from said absorption measurement the amount of HDL-C, LDL-C, VLDL-C and total cholesterol present in said test sample.

**25.** The method of Claim 24 wherein said zinc perchlorate is a zinc perchlorate hydrate.

**26.** The method of Claim 25 wherein said perchlorate is zinc perchlorate hexahydrate and said acyl compound is acetyl chloride.

**27.** The method of Claim 24 wherein said perchlorate is perchloric acid and R is lower alkyl.

**28.** The method of Claim 27 wherein R is methyl.

**29.** The method of Claim 24 wherein said perchlorate is present in a concentration of 0.3-0.7 molar in 90-100% acyl compound.

**30.** The method of Claim 29 wherein said perchlorate is present in a concentration of 0.4-0.6 molar in 95-99% acyl compound.

**31.** The method of claim 29 wherein said perchlorate is present in a concentration of 0.5 molar in 98% acyl compound.

**32.** The method of Claim 31 wherein said perchlorate is zinc perchlorate hexahydrate and said acyl compound is acetyl chloride.

**33.** The method of Claim 31 wherein said perchlorate is perchloric acid and said acyl compound is acetyl chloride.

**34.** The method of Claim 24 wherein said spectrophotometric absorption of said product is measured at wavelengths from between 150 to 750 nm.

**35.** The method of Claim 34 wherein said spectrophotometric absorption of said product is measured at three or more distinct wavelengths from between 150 to 750 nm.

**36.** The method of Claim 35 wherein said spectrophotometric absorption of said product is measured at wavelengths of about 410 nm, about 456 nm and about 518 nm.

**37.** The method of claim 35 wherein the amounts of HDL-C, LDL-C and VLDL-C present in said sample are determined by solving an algorithm which correlates the spectrophotometric absorption of said product to the amounts of HDL-C, LDL-C and VLDL-C present in said test sample.

**38.** The method of Claim 37 wherein the amounts of HDL-C, LDL-C and VLDL-C present in said sample are determined by solving an $n \cdot n$ matrix wherein n is the number of distinct wavelengths at which the spectrophotometric absorption of said product is measured.

**39.** The method of Claim 38 wherein n is three.

**40.** The method of Claim 39 wherein said algorithm involves multivariate regression analysis.

**41.** The method of Claim 40 wherein said multivariate regression analysis comprises principal component analysis, pattern recognition analysis or partial least squares analysis.

**42.** The method of Claim 24 wherein the rate at which said spectrophotometrically active product is formed is controlled by the addition of a modifier to said acyl compound and said perchlorate.

**43.** The method of Claim 42 wherein said rate is decreased and said modifier is selected from the group consisting of water, glacial acetic acid and chloroform.

**44.** The method of Claim 42 wherein said rate is increased and said modifier is selected from the group consisting of HCl and $HClO_4$.

**45.** The method of Claim 43 wherein said modifier is present at a concentration of greater than about 10% v/v based upon said acyl compound.

**46.** The method of Claim 44 wherein said modifier is present at a concentration of about 1-2% v/v based upon said acyl compound.

**47.** A chemical reagent which comprises an acyl compound having the formula:

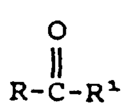

wherein $R^1$ is halogen, R is lower alkyl; and a perchlorate effective to form a spectrophotometrically active product with an analyte, said perchlorate selected from the group consisting of zinc perchlorate, barium perchlorate and perchloric acid.

**48.** The reagent of Claim 47 wherein said analyte is cholesterol and said spectrophotometrically active product is capable of detection by circular dichroism, absorption spectrophotometry, fluorescence spectrophotometry or derivative absorption spectrophotometry.

**49.** The reagent of Claim 48 wherein said zinc perchlorate is a zinc perchlorate hydrate.

**50.** The reagent of Claim 49 wherein said perchlorate is zinc perchlorate hexahydrate and said acyl compound is acetyl chloride.

**51.** The reagent of claim 47 wherein said perchlorate is perchloric acid and R is lower alkyl.

**52.** The reagent of Claim 51 wherein R is methyl.

**53.** The reagent of Claim 47 wherein said perchlorate is present in said reagent at a concentration of 0.3-0.7 molar in 90-100% acyl compound.

**54.** The reagent of Claim 53 wherein said perchlorate is present in said reagent at a concentration of 0.4-0.6 molar in 95-99% acyl compound.

**55.** The reagent of Claim 54 wherein said perchlorate is present in said reagent at a concentration of 0.5 molar in 98% acyl compound.

**56.** The reagent of Claim 47 further comprising a modifier to control the rate of reaction at which said spectrophotometrically active product is formed.

**57.** The reagent of Claim 56 wherein said rate is decreased and said modifier is selected from the group consisting of water, glacial acetic acid and chloroform.

**58.** The reagent of Claim 57 wherein said modifier is present in said reagent at a concentration of greater than about 10% v/v based upon said acyl compound.

**59.** The reagent of Claim 56 wherein said rate is increased and said modifier is selected from the group consisting of HCl and $HClO_4$.

**60.** The reagent of Claim 59 wherein said modifier is present at a concentration of about 1-2% v/v based upon said acyl compound.

**61.** A spectrophotometer for the direct, simultaneous quantitative determination of total cholesterol and its distribution among HDL-C, LDL-C, VLDL-C subfractions in a test sample which comprises means for measuring the spectrophotometric activity of a spectrophotometrically active product formed by contacting cholesterol in a test sample with an acyl compound having the formula:

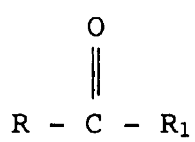

wherein $R_1$ is halogen, R is lower alkyl; and a perchlorate effective to form said product with said cholesterol, said perchlorate selected from the group consisting of zinc perchlorate, barium perchlorate and perchloric acid; and means for determining from said spectrophotometric activity the amount of total cholesterol and HDL-C, LDL-C and VLDL-C present in said test sample.

**62.** The spectrophotometer of Claim 61 wherein said means for measuring includes the capability of measuring the spectrophotometric absorption of said product at wavelengths from between 150 nm to 700 nm.

**63.** The spectrophotometer of Claim 62 wherein said means for measuring includes the capability of measuring said absorption spectrum at three or more distinct wavelengths from between 150 nm to 700 nm.

**64.** The spectrophotometer of Claim 63 wherein said absorption is measured at wavelengths of about 410 nm, about 456 nm and about 518 nm.

**65.** The spectrophotometer of Claim 61 wherein said means for determining the amount of total cholesterol, HDL-C, LDL-C and VLDL-C present in said test sample solves an algorithm which correlates the spectrophotometric activity of said product to the amount of total cholesterol, HDL-C, LDL-C and VLDL-C present in said sample.

**66.** The spectrophotometer of Claim 65 wherein said means for determining solves an $n \cdot n$ matrix wherein n is the number of distinct wavelengths at which the spectrophotometric activity of said product is measured.

**67.** The spectrophotometer of Claim 66 wherein n is three.

**68.** The spectrophotometer of Claim 65 wherein said algorithm involves multivariate regression analysis.

**69.** The spectrophotometer of Claim 68 wherein said multivariate regression analysis comprises principal component analysis, pattern recognition analysis or partial least squares analysis.

**70.** The spectrophotometer of Claim 61 further comprising means for adding said acyl compound and said perchlorate to said test sample.

**Patentansprüche**

**1.** Verfahren zur Bildung eines spektrophotometrisch aktiven Produkts von Cholesterol, umfassend das in-Kontaktbringen von Cholesterol mit einer Acyl-Verbindung der Formel:

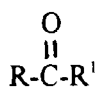

worin $R^1$ ein Halogen ist, R ein Niederalkylrest ist; und einem Perchlorat, das wirksam ist, ein spektrophotometrisch aktives Produkt des Cholesterols zu bilden, wobei das besagte Perchlorat aus der Gruppe bestehend aus Zinkperchlorat, Bariumperchlorat und Perchlorsäure ausgewählt wird.

**2.** Verfahren nach Anspruch 1, worin das Cholesterol unter den HDL-C, LDL-C und VLDL-C-Unterfraktionen verteilt ist.

**3.** Verfahren nach Anspruch 1, worin das Perchlorat Zinkperchlorat-Hexahydrat ist.

**4.** Verfahren nach Anspruch 1, worin $R^1$ Chlor ist und R eine Methylgruppe ist.

**5.** Verfahren nach Anspruch 1, worin das Perchlorat in einer 0,7 molaren Konzentration in 90-100%-iger Acyl-Verbindung vorliegt.

**6.** Verfahren nach Anspruch 5, worin das Perchlorat in einer 0,4 - 0,6 molaren Konzentration in einer 95 -99%igen Konzentration der Acyl-Verbindung vorliegt.

**7.** Verfahren nach Anspruch 6, worin das Perchlorat in einer 0,5 molaren Konzentration in 98%-iger Acyl-Verbindung vorliegt.

**8.** Verfahren nach Anspruch 7, worin das Perchlorat Zinkperchlorat-Hexahydrat ist und die Acyl-Verbindung Acetylchlorid ist.

**9.** Verfahren nach Anspruch 7, worin das Perchlorat Perchlorsäure ist und die Acyl-Verbindung Acetylchlorid ist.

**10.** Verfahren nach Anspruch 1, worin das spektrophotometrisch aktive Produkt ein farbiges Produkt ist.

**11.** Verfahren zur Bestimmung der Menge an in einer Testprobe vorhandenem Cholesterol, umfassend das in-Kontaktbringen einer Testprobe, in welcher Cholesterol vorliegt, mit einer Acyl-Verbindung der Formel:

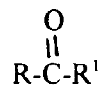

worin $R^1$ ein Halogen ist, R ein Niederalkylrest ist; und einem Perchlorat, das wirksam ist, ein spektrophotometrisch aktives Produkt des Cholesterols zu bilden, wobei das Perchlorat aus der Gruppe bestehend aus Zinkperchlorat, Bariumperchlorat und Perchlorsäure ausgewählt wird, und Auswerten der spektrophotometrischen Aktivität, um die Menge an in der Probe vorhandenem Cholesterol zu bestimmen.

**12.** Verfahren nach Anspruch 11, worin das Cholesterol unter den HDL-C, LDL-C und VLDL-C-Unterfraktionen verteilt ist.

**13.** Verfahren nach Anspruch 12, worin das Perchlorat Perchlorsäure ist und R ein Niederalkylrest ist.

**14.** Verfahren nach Anspruch 13, worin R eine Methylgruppe ist.

**15.** Verfahren nach Anspruch 12, worin das Zinkperchlorat Zinkperchlorat-Hydrat ist und R ein Niederalkylrest ist.

**16.** Verfahren nach Anspruch 15, worin das Perchlorat Zinkperchlorat-Hexahydrat ist und die Acyl-Verbindung Acetylchlorid ist.

**17.** Verfahren nach Anspruch 11, worin das Perchlorat in einer 0,3 - 0,7 molaren Konzentration in 90-100%-iger Acyl-Verbindung vorliegt.

**18.** Verfahren nach Anspruch 17, worin das Perchlorat in einer 0,4 - 0,6 molaren Konzentration in 95 -99%-iger Acyl-Verbindung vorliegt.

**19.** Verfahren nach Anspruch 18, worin das Perchlorat in einer 0,5 molaren Konzentration in 98%-iger Acyl-Verbindung vorliegt.

**20.** Verfahren nach Anspruch 19, worin das Perchlorat Zinkperchlorat-Hexahydrat ist und die Acyl-Verbindung Acetylchlorid ist.

**21.** Verfahren nach Anspruch 19, worin das Perchlorat Perchlorsäure ist und die Acyl-Verbindung Acetylchlorid ist.

**22.** Verfahren nach Anspruch 11, worin das spektrophotometrisch aktive Produkt zum Nachweis durch Zirkulardichroismus, Absorptionsspektrophotometrie, Fluoreszenzspektrophotometrie oder Derivat-Absorptionsspektrophotometrie fähig ist.

**23.** Verfahren nach Anspruch 11, worin die spektrophotometrische Wirkung durch Messen des Absorptionsspektrums des Produkts bei Wellenlängen zwischen 150 bis 750 nm ausgewertet wird.

**24.** Verfahren zur direkten, gleichzeitigen, quantitativen Bestimmung des Gesamtcholesterols und seiner Verteilung in den HDL-C, LDL-C und VLDL-C Unterfraktionen in einer Testprobe, umfassend das in-Kontakt-bringen einer Testprobe mit Cholesterol in HDL-C, LDL-C und VLDL-C Unterfraktionen mit einer Acylkomponente mit der Formel:

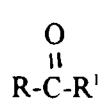

worin $R^1$ ein Halogen ist, R ein Niederalkylrest ist; und einem Perchlorat, das wirksam ist, ein spektrophotometrisch aktives Produkt des Cholesterols zu bilden, wobei das Perchlorat aus der Gruppe bestehend aus Zinkperchlorat, Bariumperchlorat und Perchlorsäure ausgewählt wird; Messen der spektrophotometrischen Absorption des Produkts; und Bestimmen aus der Absorptionsmessung der Mengen an HDL-C, LDL-C, VLDL-C und Gesamtcholesterol, die in der Testprobe vorliegen.

**25.** Verfahren nach Anspruch 24, worin Zinkperchlorat ein Zinkperchlorat-Hexahydrat ist.

**26.** Verfahren nach Anspruch 25, worin das Perchlorat Zinkperchlorat-Hexahydrat ist und die Acyl-Verbindung Acetylchlorid ist.

**27.** Verfahren nach Anspruch 24, worin das Perchlorat Perchlorsäure ist und R ein Niederalkylrest ist.

**28.** Verfahren nach Anspruch 27, worin R eine Methylgruppe ist.

**29.** Verfahren nach Anspruch 24, worin das Perchlorat in einer 0,3 - 0,7 molaren Konzentration in 90-100%-iger Acyl-Verbindung vorliegt.

**30.** Verfahren nach Anspruch 29, worin das Perchlorat in einer 0,4 - 0,6 molaren Konzentration in 95 -99%-iger Acyl-Verbindung vorliegt.

**31.** Verfahren nach Anspruch 29, worin das Perchlorat in einer 0,5 molaren Konzentration in 98%-iger Acyl-Verbindung vorliegt.

**32.** Verfahren nach Anspruch 31, worin das Perchlorat Zinkperchlorat-Hexahydrat ist und die Acyl-Verbindung Acetylchlorid ist.

**33.** Verfahren nach Anspruch 31, worin das Perchlorat Perchlorsäure ist und die Acyl-Verbindung Acetylchlorid ist.

**34.** Verfahren nach Anspruch 24, worin die spektrophotometrische Absorption des Produkts bei Wellenlängen von 150 bis 750 nm gemessen wird.

**35.** Verfahren nach Anspruch 34, worin die spektrophotometrische Absorption des Produkts bei drei oder mehr bestimmten Wellenlängen zwischen 150 und 750 nm gemessen wird.

**36.** Verfahren nach Anspruch 35, worin die spektrophotometrische Absorption des Produkts bei Wellenlängen von etwa 410 nm, etwa 456 nm und etwa 518 nm gemessen wird.

**37.** Verfahren nach Anspruch 35, worin die in der Probe vorhandenen HDL-C, LDL-C und VLDL-C- Mengen durch Lösen eines Algorithmus, der die spektrophotometrische Absorption des Produkts zu den HDL-C, LDL-C und VLDL-C-Mengen in Beziehung setzt, bestimmt werden.

**38.** Verfahren nach Anspruch 37, worin die in der Probe vorhandenen HDL-C, LDL-C und VLDL-C- Mengen durch Lösen einer n n Matrix, worin n die Zahl der einzelnen Wellenlängen ist, bei der die spektrophotometrische Absorption des Produkts gemessen wird, bestimmt werden.

**39.** Verfahren nach Anspruch 38, worin n drei ist.

**40.** Verfahren nach Anspruch 39, worin der Algorithmus die multivariante Regressionsanalyse einschließt.

**41.** Verfahren nach Anspruch 40, worin die multivariante Regressionsanalyse Hauptkomponentenanalyse, Bilderkennungsanalyse oder Partialanalyse der kleinsten Quadrate umfaßt.

**42.** Verfahren nach Anspruch 24, worin die Geschwindigkeit, bei der das spektrophotometrisch aktive Produkt geformt wird, durch die Zugabe eines Modifikators zur Acyl-Verbindung und zum Perchlorat gesteuert wird.

**43.** Verfahren nach Anspruch 42, worin die Geschwindigkeit herabgesetzt wird und der Modifikator aus der Gruppe bestehend aus Wasser, Eisessig und Chloroform ausgewählt ist.

**44.** Verfahren nach Anspruch 42, worin die Geschwindigkeit erhöht wird und der Modifikator aus der Gruppe bestehend aus HCl und $HClO_4$ ausgewählt ist.

**45.** Verfahren nach Anspruch 43, worin der Modifikator in einer Konzentration größer als etwa 10 Vol.-%, basierend auf der Acyl-Komponente, vorliegt.

**46.** Verfahren nach Anspruch 44, worin der Modifikator in einer Konzentration von etwa 1 - 2 Vol.-%, basierend auf der Acyl-Komponente, vorliegt.

**47.** Chemisches Reagenz, umfassend eine Acyl-Verbindung mit der Formel:

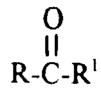

worin $R^1$ ein Halogen ist, R ein Niederalkylrest ist; und ein Perchlorat, das wirksam ist, ein spektrophotometrisch aktives Produkt mit einem zu bestimmenden Stoffzu bilden, wobei das Perchlorat aus der Gruppe bestehend aus Zinkperchlorat, Bariumperchlorat und Perchlorsäure ausgewählt wird.

**48.** Reagenz nach Anspruch 47, worin der zu bestimmende Stoff Cholesterol ist und das spektrophotometrisch aktive Produkt zur Bestimmung durch Zirkulardichroismus, Absorptionsspektrophotometrie, Fluoreszenzspektrophotometrie oder Derivatabsorptionsphotospektrometrie fähig ist.

**49.** Reagenz nach Anspruch 48, worin das Zinkperchlorat Zinkperchlorat-Hexahydrat ist.

**50.** Reagenz nach Anspruch 49, worin das Perchlorat Zinkperchlorat-Hexahydrat ist und die Acyl-Verbindung Acetylchlorid ist.

**51.** Reagenz nach Anspruch 47, worin das Perchlorat Perchlorsäure ist und R ein Niederalkylrest ist.

**52.** Reagenz nach Anspruch 51, worin R eine Methylgruppe ist.

**53.** Reagenz nach Anspruch 47, worin das Perchlorat im Reagenz in einer 0,3 - 0,7 molaren Konzentration in 90-100%-iger Acyl-Verbindung vorliegt.

**54.** Reagenz nach Anspruch 53, worin das Perchlorat im Reagenz in einer 0,4 - 0,6 molaren Konzentration in 95 -99%iger Acyl-Verbindung vorliegt.

**55.** Reagenz nach Anspruch 54, worin das Perchlorat im Reagenz in einer 0,5 molaren Konzentration in 98%iger Acyl-Verbindung vorliegt.

**56.** Reagenz nach Anspruch 47, zusätzlich umfassend einen Modifikator zur Steuerung der Reaktionsgeschwindigkeit, bei der das spektrophotometrisch aktive Produkt geformt wird.

**57.** Reagenz nach Anspruch 56, worin die Geschwindigkeit herabgesetzt ist und der Modifikator aus der Gruppe bestehend aus Wasser, Eisessig und Chloroform ausgewählt ist.

**58.** Reagenz nach Anspruch 57, worin der Modifikator im Reagenz in einer Konzentration größer als etwa 10 Vol.-%, basierend auf der Acyl-Komponente, vorliegt.

**59.** Reagenz nach Anspruch 56, worin die Geschwindigkeit erhöht ist und der Modifikator aus der Gruppe bestehend aus HCl und $HClO_4$ ausgewählt ist.

**60.** Reagenz nach Anspruch 59, worin der Modifikator in einer Konzentration von etwa 1 - 2 Vol.-%, basierend auf der Acyl-Komponente, vorliegt.

**61.** Spektrophotometer für die direkte, gleichzeitige, quantitative Bestimmung des Gesamtcholesterols und seiner Verteilung in den HDL-C, LDL-C und VLDL-C Unterfraktionen in einer Testprobe, umfassend Mittel zur Messung der spektrophotometrischen Aktivität eines spektrophotometrisch aktiven Produkts, geformt durch in-Kontakt-bringen von Cholesterol in einer Testprobe mit einer Acyl-Verbindung der Formel :

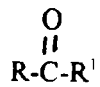

worin $R^1$ ein Halogen ist, R Niederalkyl ist; und einem Perchlorat, das wirksam ist, ein Produkt mit dem Cholesterol zu formen, wobei das Perchlorat aus der Gruppe bestehend aus Zinkperchlorat, Bariumperchlorat und Perchlorsäure ausgewählt ist; und Mittel zur Bestimmung aus der spektrophotometrischen Wirkung der Menge an Gesamt- und HDL-C-, LDL-C- und VLDL-C-Cholesterol in der Testprobe.

**62.** Spektrophotometer nach Anspruch 61, worin die Mittel zur Messung die Fähigkeit zur Messung der spektrophotometrischen Absorption bei Wellenlängen von zwischen 150 nm und 700 nm einschließen.

**63.** Spektrophotometer nach Anspruch 62, worin die Mittel zur Messung die Fähigkeit zur Messung des Absorptionsspektrums bei drei oder mehr bestimmten Wellenlängen zwischen 150 nm und 700 nm einschließen.

**64.** Spektrophotometer nach Anspruch 63, worin die Absorption bei Wellenlängen von etwa 410 nm, etwa 456 nm und etwa 518 nm gemessen wird.

**65.** Spektrophotometer nach Anspruch 61, worin die Mittel zur Bestimmung der Menge an in der Testprobe vorhandenem Gesamtcholesterol, HDL-C, LDL-C und VLDL-C einen Algorithmus löst, der die spektrophotometrische Aktivität des Produkts mit der Menge an Gesamtcholesterol, HDL-C, LDL-C und VLDL-C in Beziehung setzt.

**66.** Spektrophotometer nach Anspruch 65, worin die Mittel zur Bestimmung eine n n Matrix, worin n die Zahl der einzelnen Wellenlängen ist, bei der die spektrophotometrische Absorption des Produkts gemessen wird, löst.

**67.** Spektrophotometer nach Anspruch 66, worin n drei ist.

**68.** Spektrophotometer nach Anspruch 65, worin der Algorithmus die multivariante Regressionsanalyse einschließt.

**69.** Spektrophotometer nach Anspruch 68, worin die multivariante Regressionsanalyse Hauptkomponentenanalyse, Bilderkennungsanalyse oder Partialanalyse der kleinsten Quadrate umfaßt.

**70.** Spektrophotometer nach Anspruch 61, zusätzlich umfassend Mittel zur Zugabe der Acyl-Verbindung und des Perchlorats zur Testprobe.

## Revendications

**1.** Procédé pour former un produit de cholestérol à activité spectrophotométrique, qui comprend la mise en contact de cholestérol avec un composé acylé ayant la formule :

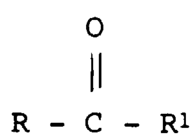

dans laquelle $R^1$ est un halogène, R est un radical alkyle inférieur ; et un perchlorate à même de former un produit à activité spectrophotométrique dudit cholestérol, ledit perchlorate étant choisi dans le groupe constitué du perchlorate de zinc, du perchlorate de baryum et de l'acide perchlorique.

**2.** Procédé selon la revendication 1, dans lequel ledit cholestérol est réparti parmi les sous-fractions HDL-C, LDL-C et VLDL-C.

**3.** Procédé selon la revendication 1, dans lequel ledit perchlorate est le perchlorate de zinc hexahydraté.

**4.** Procédé selon la revendication 1, dans lequel $R^1$ est le chlore et R est le groupe méthyle.

**5.** Procédé selon la revendication 1, dans lequel ledit perchlorate est présent selon une concentration de 0,3 - 0,7 molaire dans le composé acylé à 90 - 100 %.

**6.** Procédé selon la revendication 5, dans lequel ledit perchlorate est présent selon une concentration de 0,4 à 0,6 molaire dans le composé acylé à 95 - 99 %.

**7.** Procédé selon la revendication 6, dans lequel ledit perchlorate est présent selon une concentration de 0,5 molaire dans le composé acylé à 98 %.

**8.** Procédé selon la revendication 7, dans lequel ledit perchlorate est le perchlorate de zinc hexahydraté, et ledit composé acylé est le chlorure d'acétyle.

**9.** Procédé selon la revendication 7, dans lequel ledit perchlorate est l'acide perchlorique, et ledit composé acylé est le chlorure d'acétyle.

**10.** Procédé selon la revendication 1, dans lequel ledit produit à activité spectrophotométrique est un produit coloré.

**11.** Procédé pour déterminer la quantité de cholestérol présente dans un échantillon d'essai, qui comprend la mise en contact d'un échantillon d'essai dans lequel le cholestérol est présent avec un composé acylé ayant la formule :

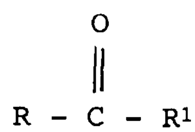

dans laquelle $R^1$ est un halogène, R est un radical alkyle inférieur, et un perchlorate à même de former un produit à activité spectrophotométrique avec ledit cholestérol, ledit perchlorate étant choisi dans le groupe constitué du perchlorate de zinc, du perchlorate de baryum et de l'acide perchlorique, et à évaluer ladite activité spectrophotométrique pour déterminer la quantité dudit cholestérol présente dans ledit échantillon.

**12.** Procédé selon la revendication 11, dans lequel ledit cholestérol est réparti parmi les sous-fractions HDL-C, LDL-C et VLDL-C.

**13.** Procédé selon la revendication 12, dans lequel ledit perchlorate est l'acide perchlorique, et R est un radical alkyle inférieur.

**14.** Procédé selon la revendication 13, dans lequel R est le groupe méthyle.

**15.** Procédé selon la revendication 12, dans lequel ledit perchlorate de zinc est un perchlorate de zinc hydraté, et R est un radical alkyle inférieur.

**16.** Procédé selon la revendication 15, dans lequel ledit perchlorate est le perchlorate de zinc hexahydraté, et ledit composé acylé est le chlorure d'acétyle.

**17.** Procédé selon la revendication 11, dans lequel ledit perchlorate est présent selon une concentration de 0,3 -0,7 molaire dans le composé acylé à 90 - 100 %.

**18.** Procédé selon la revendication 17, dans lequel ledit perchlorate est présent selon une concentration de 0,4 - 0,6 molaire dans le composé acylé à 95 - 99 %.

**19.** Procédé selon la revendication 18, dans lequel ledit perchlorate est présent selon une concentration de 0,5 molaire dans le composé acylé à 98 %.

**20.** Procédé selon la revendication 19, dans lequel ledit perchlorate est le perchlorate de zinc hexahydraté, et ledit composé acylé est le chlorure d'acétyle.

**21.** Procédé selon la revendication 19, dans lequel ledit perchlorate est l'acide perchlorique, et ledit composé acylé

est le chlorure d'acétyle.

**22.** Procédé selon la revendication 11, dans lequel ledit produit à activité spectrophotométrique peut être détecté par dichroïsme circulaire, spectrophotométrie d'absorption, spectrophotométrie par fluorescence ou spectrophotométrie d'absorption dérivée.

**23.** Procédé selon la revendication 11, dans lequel ladite activité spectrophotométrique est évaluée par mesure du spectre d'absorption dudit produit à des longueurs d'ondes comprises entre 150 et 750 nm.

**24.** Procédé de dosage direct simultané et quantitatif du cholestérol total, et de détermination de sa répartition parmi les sous-fractions HDL-C, LDL-C et VLDL-C dans un échantillon d'essai, qui comprend la mise en contact d'un échantillon d'essai contenant un cholestérol réparti parmi les sous-fractions HDL-C, LDL-C et VLDL-C, avec un composé acylé ayant la formule :

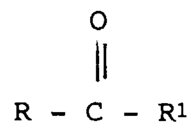

dans laquelle $R^1$ est un halogène, R est un radical alkyle inférieur ; et un perchlorate à même de former un produit à activité spectrophotométrique avec ledit cholestérol, ledit perchlorate étant choisi dans le groupe constitué du perchlorate de zinc, du perchlorate de baryum et de l'acide perchlorique ; à mesurer l'absorption spectrophotométrique dudit produit ; et à calculer à partir de ladite mesure de l'absorption la quantité de HDL-C, de LDL-C, de VLDL-C et de cholestérol total, présente dans ledit échantillon d'essai.

**25.** Procédé selon la revendication 24, dans lequel ledit perchlorate de zinc est un perchlorate de zinc hydraté.

**26.** Procédé selon la revendication 25, dans lequel ledit perchlorate est le perchlorate de zinc hexahydraté, et ledit composé acylé est le chlorure d'acétyle.

**27.** Procédé selon la revendication 24, dans lequel ledit perchlorate est l'acide perchlorique, et R est un radical alkyle inférieur.

**28.** Procédé selon la revendication 27, dans lequel R est le groupe méthyle.

**29.** Procédé selon la revendication 24, dans lequel ledit perchlorate est présent selon une concentration de 0,3 - 0,7 molaire dans le composé acylé à 90 - 100 %.

**30.** Procédé selon la revendication 29, dans lequel ledit perchlorate est présent selon une concentration de 0,4 - 0,6 molaire dans le composé acylé à 95 - 99 %.

**31.** Procédé selon la revendication 29, dans lequel ledit perchlorate est présent selon une concentration de 0,5 molaire dans le composé acylé à 98.

**32.** Procédé selon la revendication 31, dans lequel ledit perchlorate est le perchlorate de zinc hexahydraté, et ledit composé acylé est le chlorure d'acétyle.

**33.** Procédé selon la revendication 31, dans lequel ledit perchlorate est l'acide perchlorique, et ledit composé acylé est le chlorure d'acétyle.

**34.** Procédé selon la revendication 24, dans lequel ladite absorption spectrophotométrique dudit produit est mesurée à des longueurs d'ondes comprises entre 150 et 750 nm.

**35.** Procédé selon la revendication 34, dans lequel ladite absorption spectrophotométrique dudit produit est mesurée à 3 longueurs d'ondes distinctes ou plus, comprises entre 150 et 750 nm.

**36.** Procédé selon la revendication 35, dans lequel ladite absorption spectrophotométrique dudit produit est mesurée

aux longueurs d'ondes d'environ 410 nm, d'environ 456 nm et d'environ 518 nm.

**37.** Procédé selon la revendication 35, dans lequel les quantités de HDL-C, de LDL-C et de VLDL-C présentes dans ledit échantillon sont déterminées par résolution d'un algorithme qui fait une corrélation entre l'absorption spectrophotométrique dudit produit et les quantités de HDL-C, de LDL-C et de VLDL-C présentes dans ledit échantillon d'essai.

**38.** Procédé selon la revendication 37, dans lequel les quantités de HDL-C, de LDL-C et de VLDL-C présentes dans ledit échantillon sont déterminées par résolution d'une matrice n x n, où n est le nombre de longueurs d'ondes distinctes auxquelles est mesurée l'absorption spectrophotométrique dudit produit.

**39.** Procédé selon la revendication 38, dans lequel n vaut trois.

**40.** Procédé selon la revendication 39, dans lequel ledit algorithme implique une analyse par régression à plusieurs variables.

**41.** Procédé selon la revendication 40, dans lequel ladite analyse par régression à plusieurs variables comprend une analyse des composants principaux, une analyse de reconnaissance de forme ou une analyse partielle par les moindres carrés.

**42.** Procédé selon la revendication 24, dans lequel la vitesse à laquelle ledit produit à activité spectrophotométrique est formé est régulée par addition d'un modifiant audit composé acylé et audit perchlorate.

**43.** Procédé selon la revendication 42, dans lequel ladite vitesse est réduite et ledit modifiant est choisi dans le groupe constitué de l'eau, de l'acide acétique glacial et du chloroforme.

**44.** Procédé selon la revendication 42, dans lequel ladite vitesse est augmentée, et ledit modifiant est choisi dans le groupe constitué de HCL et de $HCLO_4$.

**45.** Procédé selon la revendication 43, dans lequel ledit modifiant est présent selon une concentration supérieure à environ 10 % v/v par rapport audit composé acylé.

**46.** Procédé selon la revendication 44, dans lequel ledit modifiant est présent selon une concentration d'environ 1 - 2 % v/v par rapport audit composé acylé.

**47.** Réactif chimique qui comprend un composé acylé ayant la formule :

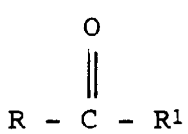

dans laquelle $R^1$ est un halogène, R est un radical alkyle inférieur ; et un perchlorate à même de former un produit à activité spectrophotométrique avec un analyte, ledit perchlorate étant choisi dans le groupe constitué du perchlorate de zinc, du perchlorate de baryum et de l'acide perchlorique.

**48.** Réactif selon la revendication 47, dans lequel ledit analyte est le cholestérol, et ledit produit à activité spectrophotométrique peut être détecté par dichroïsme circulaire, par spectrophotométrie d'absorption, par spectrophotométrie par fluorescence ou par spectrophotométrie d'absorption dérivée.

**49.** Réactif selon la revendication 48, dans lequel ledit perchlorate de zinc est un perchlorate de zinc hydraté.

**50.** Réactif selon la revendication 49, dans lequel ledit perchlorate est le perchlorate de zinc hexahydraté, et ledit composé acylé est le chlorure d'acétyle.

**51.** Réactif selon la revendication 47, dans lequel ledit perchlorate est l'acide perchlorique, et R est un radical alkyle inférieur.

**52.** Réactif selon la revendication 51, dans lequel R est le groupe méthyle.

**53.** Réactif selon la revendication 47, dans lequel ledit perchlorate est présent dans ledit réactif selon une concentration de 0,3 - 0,7 molaire dans le composé acylé à 90 - 100 %.

**54.** Réactif selon la revendication 53, dans lequel ledit perchlorate est présent dans ledit réactif selon une concentration de 0,4 - 0,6 molaire dans le composé acylé à 95 - 99 %.

**55.** Réactif selon la revendication 54, dans lequel ledit perchlorate est présent dans ledit réactif selon une concentration de 0,5 molaire dans le composé acylé à 98 %.

**56.** Réactif selon la revendication 47, qui comprend en outre un modifiant pour réguler la vitesse de réaction à laquelle est formé le produit à activité spectrophotométrique.

**57.** Réactif selon la revendication 56, dans lequel ladite vitesse est réduite, et ledit modifiant est choisi dans le groupe constitué de l'eau, de l'acide acétique glacial et du chloroforme.

**58.** Réactif selon la revendication 57, dans lequel ledit modifiant est présent dans ledit réactif selon une concentration supérieure à environ 10 % v/v par rapport audit composé acylé.

**59.** Réactif selon la revendication 56, dans lequel ladite vitesse est augmentée, et ledit modifiant est choisi dans le groupe constitué de HLC et de $HClO_4$.

**60.** Réactif selon la revendication 59, dans lequel ledit modifiant est présent selon une concentration d'environ 1 - 2 % v/v par rapport audit composé acylé.

**61.** Spectrophotomètre pour le dosage direct, simultané et quantitatif du cholestérol total, et pour déterminer sa répartition parmi les sous-fractions HDL-C, LDL-C, VLDL-C dans un échantillon d'essai, qui comprend un moyen pour mesurer l'activité spectrophotométrique d'un produit à activité spectrophotométrique formé par mise en contact de cholestérol dans un échantillon d'essai avec un composé acylé ayant la formule :

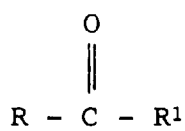

dans laquelle $R^1$ est un halogène, R est un radical alkyle inférieur ; et un perchlorate à même de former ledit produit avec ledit cholestérol, ledit perchlorate étant choisi dans le groupe constitué du perchlorate de zinc, du perchlorate de baryum et de l'acide perchlorique; et un moyen pour déterminer à partir de ladite activité spectrophotométrique la quantité de cholestérol total et de HDL-C, de LDL-C et de VLDL-C présente dans ledit échantillon d'essai.

**62.** Spectrophotomètre selon la revendication 61, dans lequel ledit moyen de mesure comprend la possibilité de mesurer l'absorption spectrophotométrique dudit produit à des longueurs d'ondes comprises entre 150 nm et 700 nm.

**63.** Spectrophotomètre selon la revendication 62, dans lequel ledit moyen de mesure comprend la possibilité de mesurer ledit spectre d'absorption à trois longueurs d'ondes distinctes ou plus, compriseS entre 150 nm et 700 nm.

**64.** Spectrophotomètre selon la revendication 63, dans lequel ladite absorption est mesurée aux longueurs d'ondes d'environ 410 nm, d'environ 456 nm et d'environ 518 nm.

**65.** Spectrophotomètre selon la revendication 61, dans lequel ledit moyen pour déterminer la quantité de cholestérol total, de HDL-C, de LDL-C et de VLDL-C présente dans ledit échantillon d'essai résout un algorithme qui fait une corrélation entre l'activité spectrophotométrique dudit produit et la quantité de cholestérol total, de HDL-C, de LDL-C et de VLDL-C présente dans ledit échantillon.

**66.** Spectrophotomètre selon la revendication 65, dans lequel ledit moyen de détermination résout une matrice n x n,

où n est le nombre de longueurs d'ondes distinctes auxquelles est mesurée l'activité spectrophotométrique dudit produit.

**67.** Spectrophotomètre selon la revendication 66, dans lequel n vaut trois.

**68.** Spectrophotomètre selon la revendication 65, dans lequel ledit algorithme implique une analyse par régression à plusieurs variables.

**69.** Spectrophotomètre selon la revendication 68, dans lequel ladite analyse par régression à plusieurs variables comprend une analyse des composants principaux, une analyse de reconnaissance de forme ou une analyse partielle des moindre carrés.

**70.** Spectrophotomètre selon la revendication 61, qui comprend en outre un moyen pour ajouter audit échantillon d'essai ledit composé acylé et ledit perchlorate.

FIG. 1

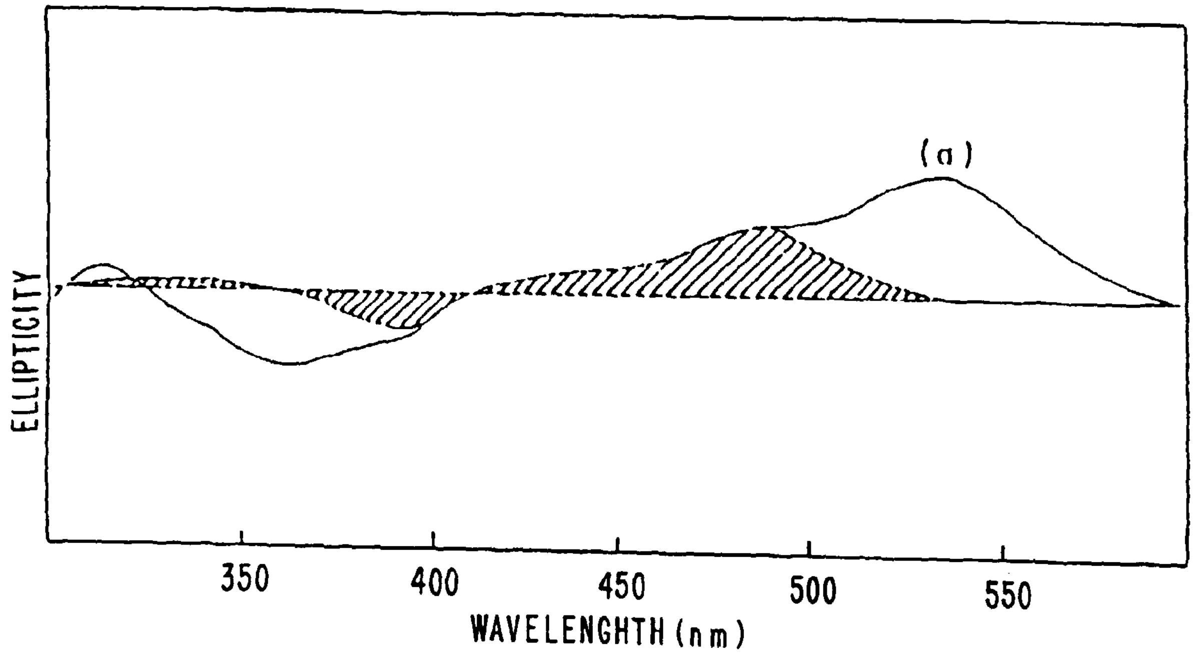
(a)
ELLIPTICITY
350
400
450
500
550
WAVELENGHTH (nm)

FIG. 2

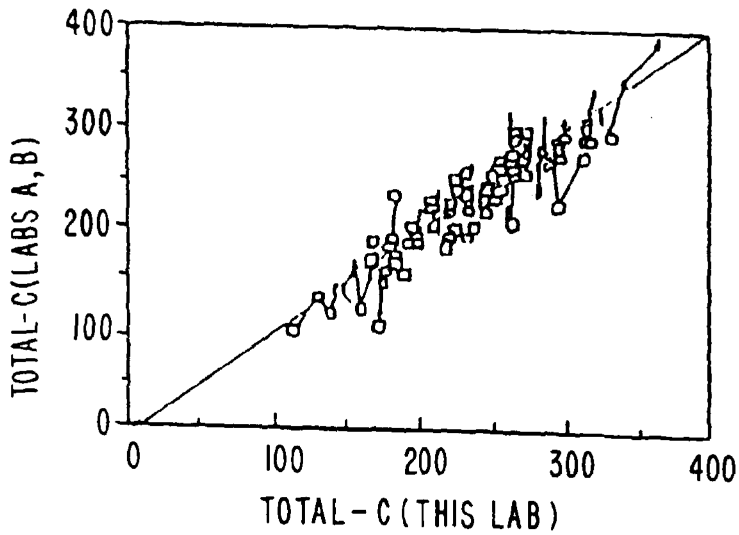
400
300
200
100
0
TOTAL-C(LABS A,B)
0
100
200
300
400
TOTAL-C (THIS LAB)

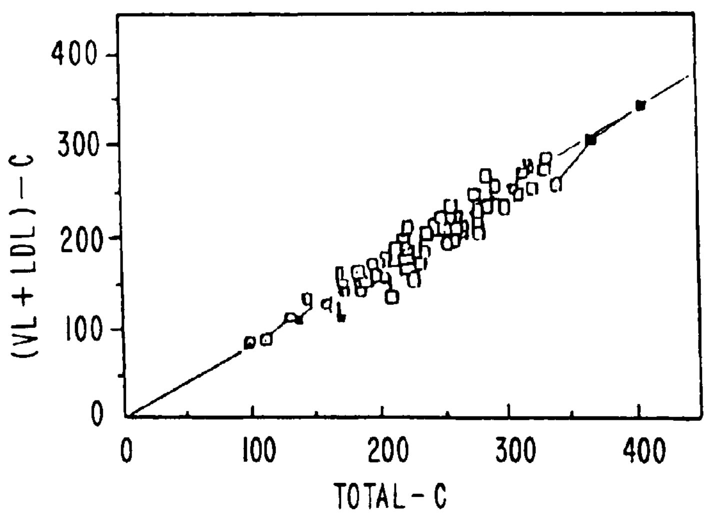


FIG. 3(A)

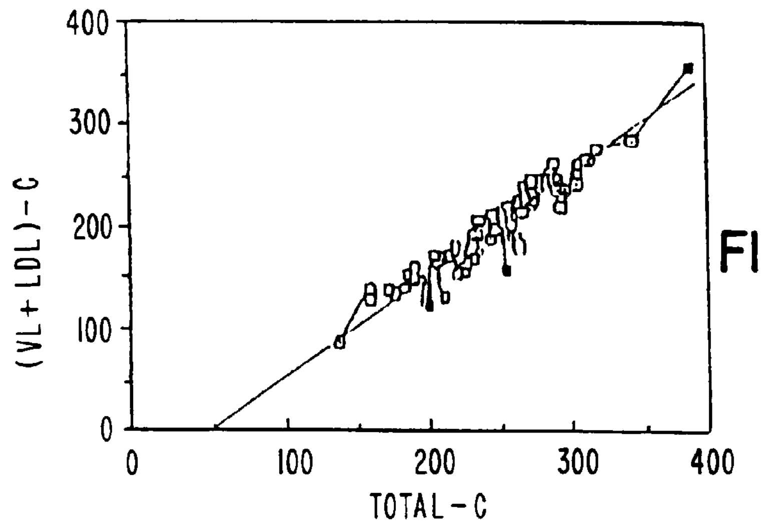


FIG. 3(B)

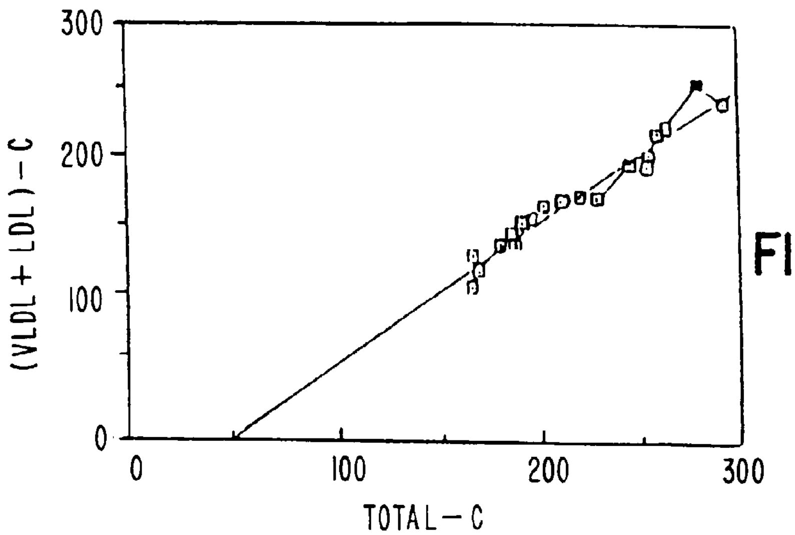


FIG. 3(C)

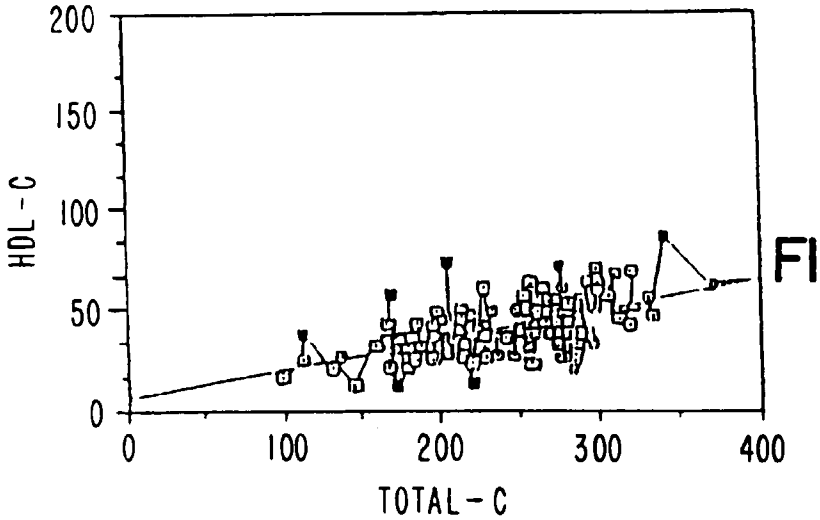


FIG. 4(A)

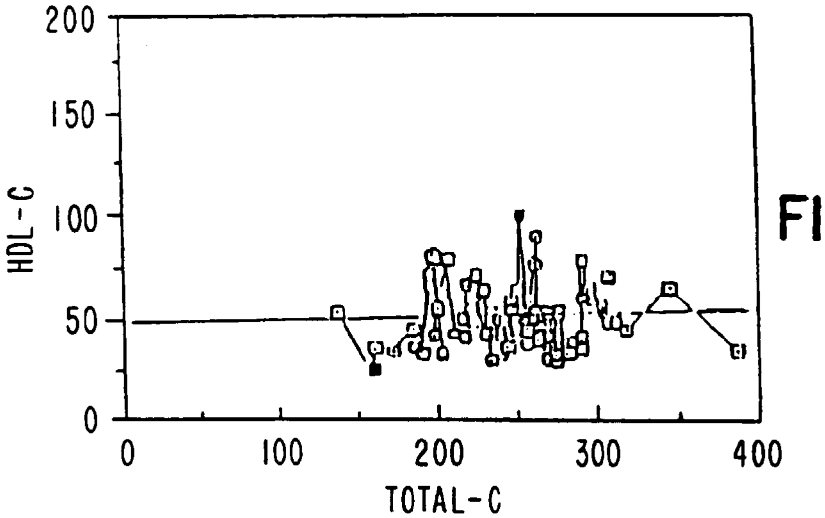


FIG. 4(B)

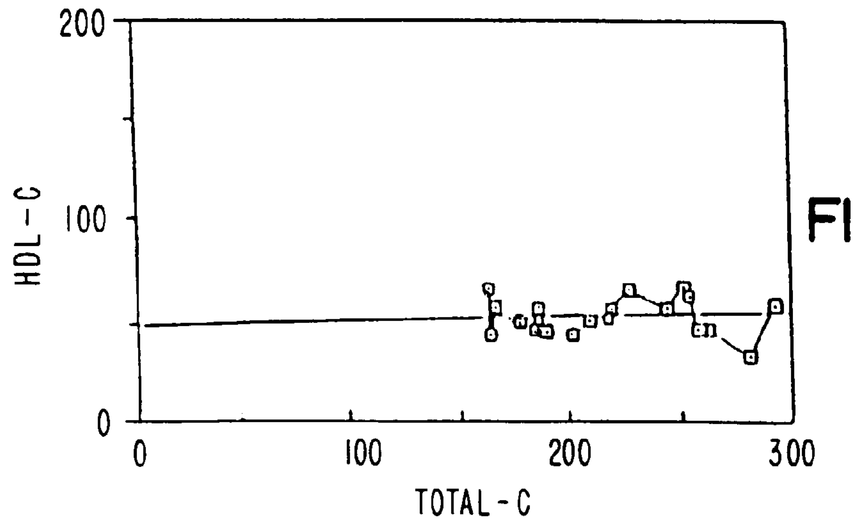


FIG. 4(C)

FIG. 5

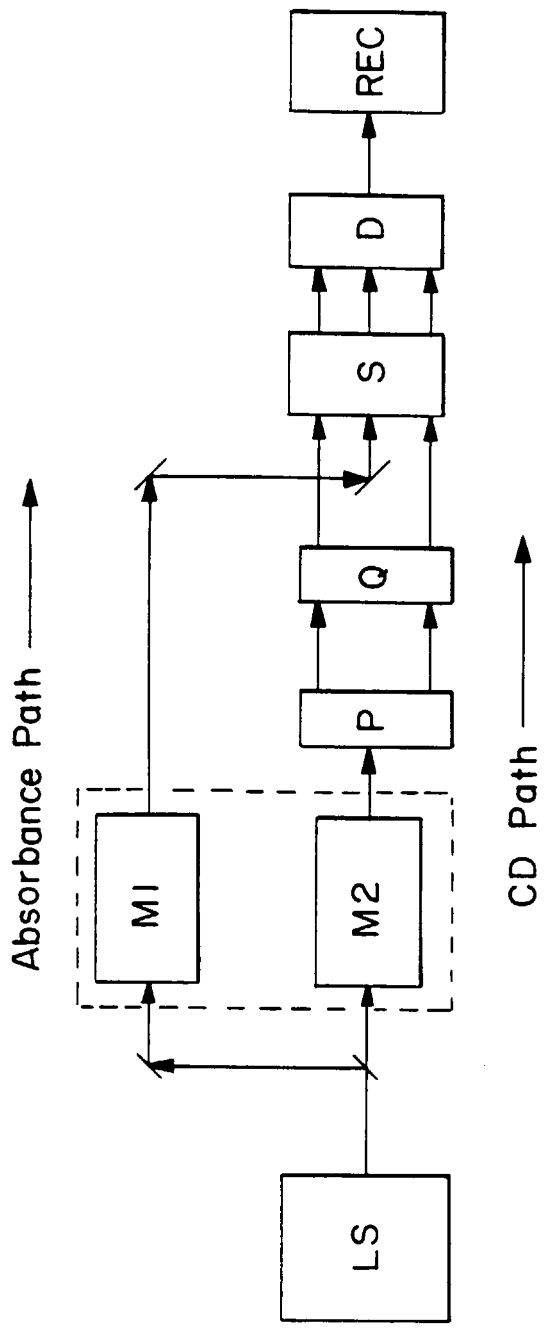

FIG. 6

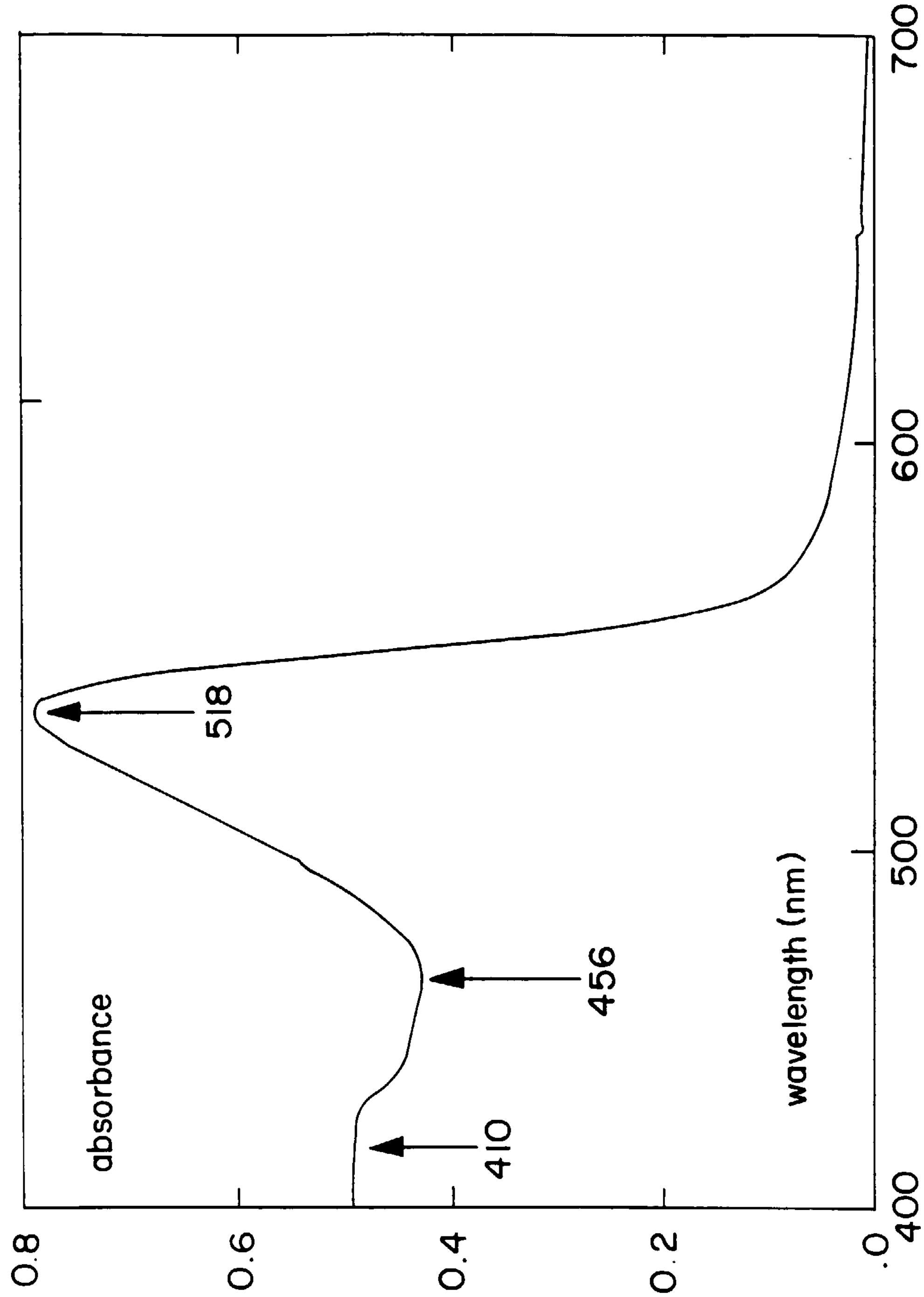
absorbance
wavelength (nm)
0.8
0.6
0.4
0.2
.0
400
500
600
700
410
456
518

FIG. 7A

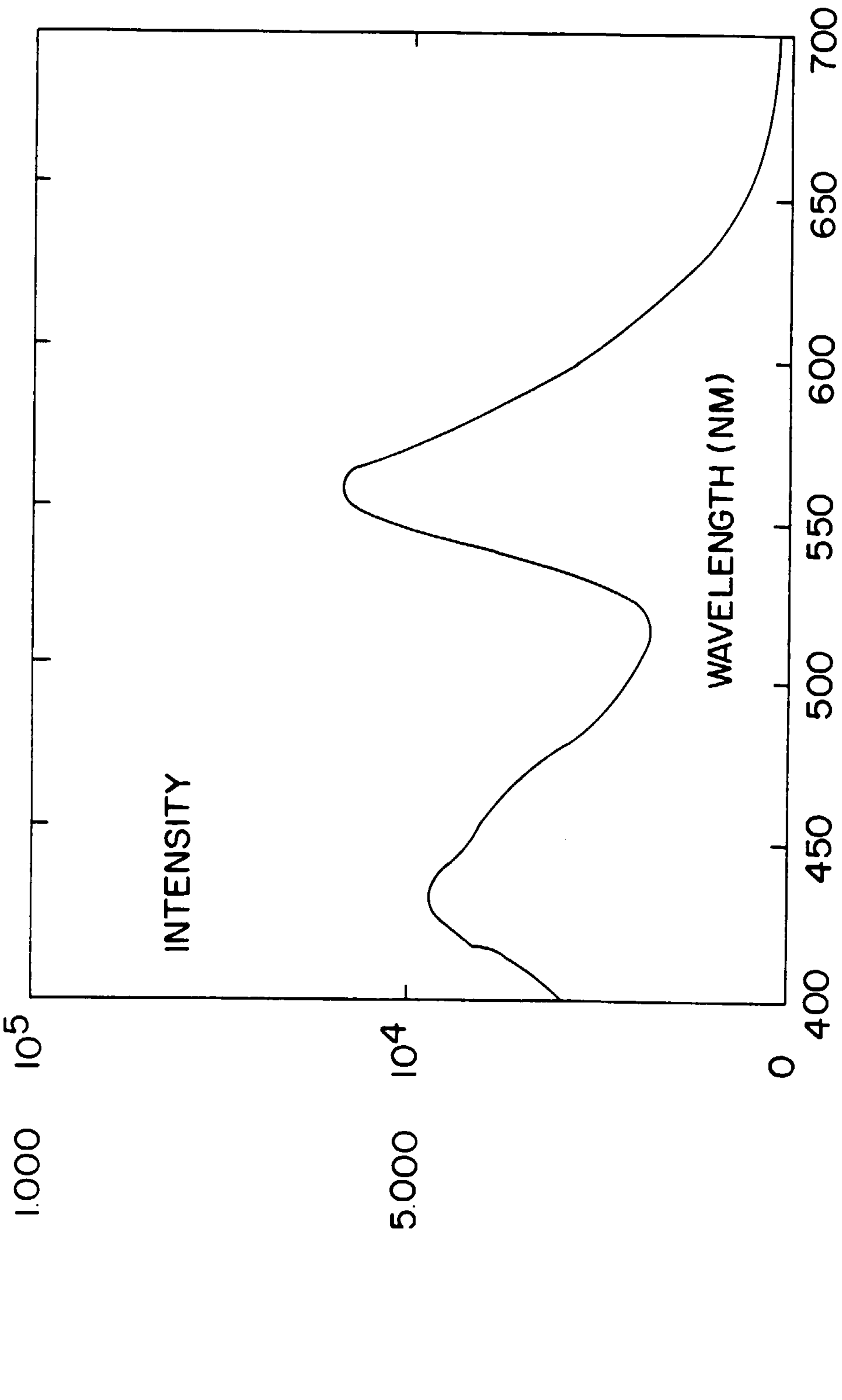
INTENSITY
WAVELENGTH (NM)
400
450
500
550
600
650
700
0
$10^4$
$10^5$
5.000
1.000

FIG. 7B

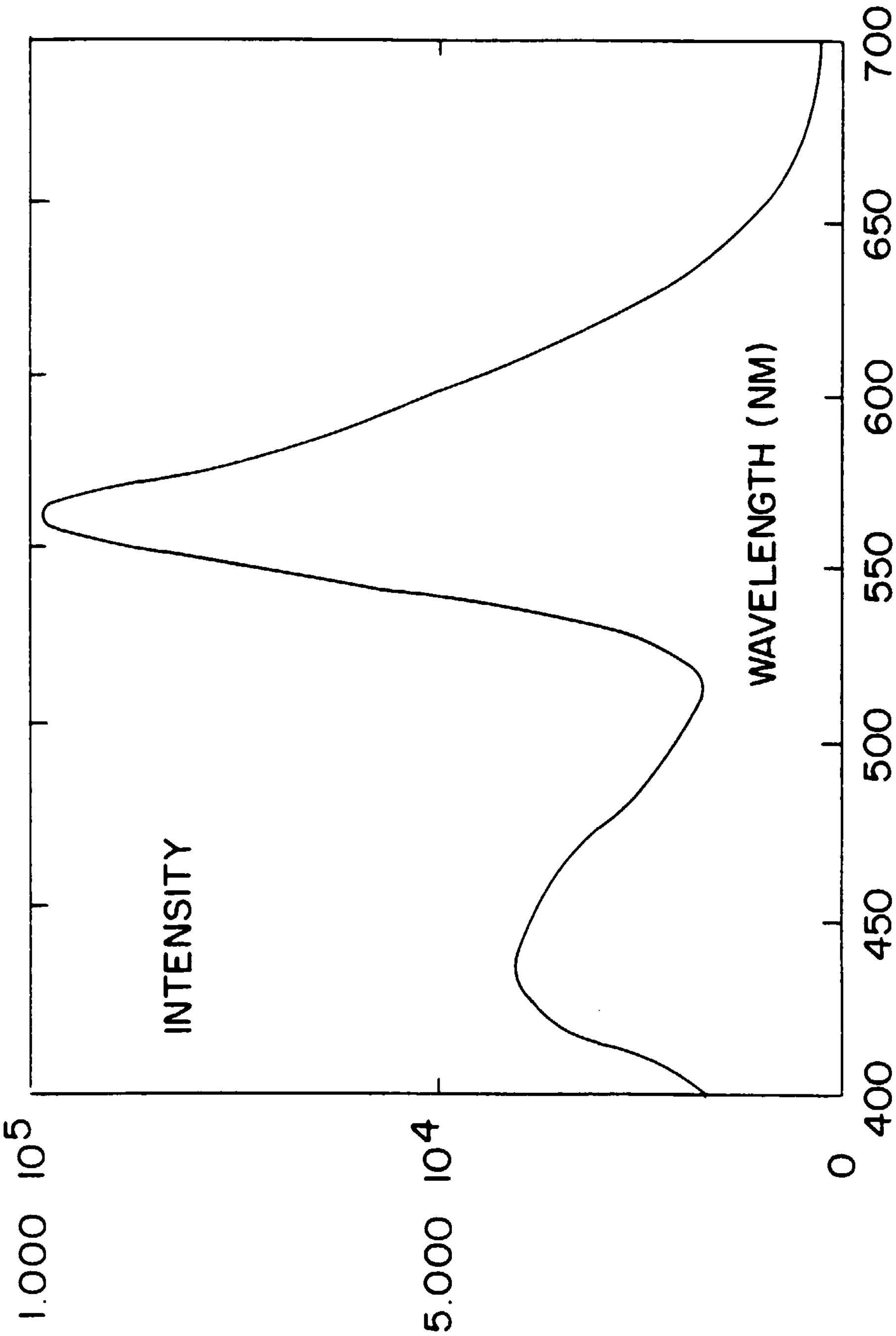
INTENSITY
WAVELENGTH (NM)
1.000 $10^5$
5.000 $10^4$
0
400
450
500
550
600
650
700

FIG. 7C

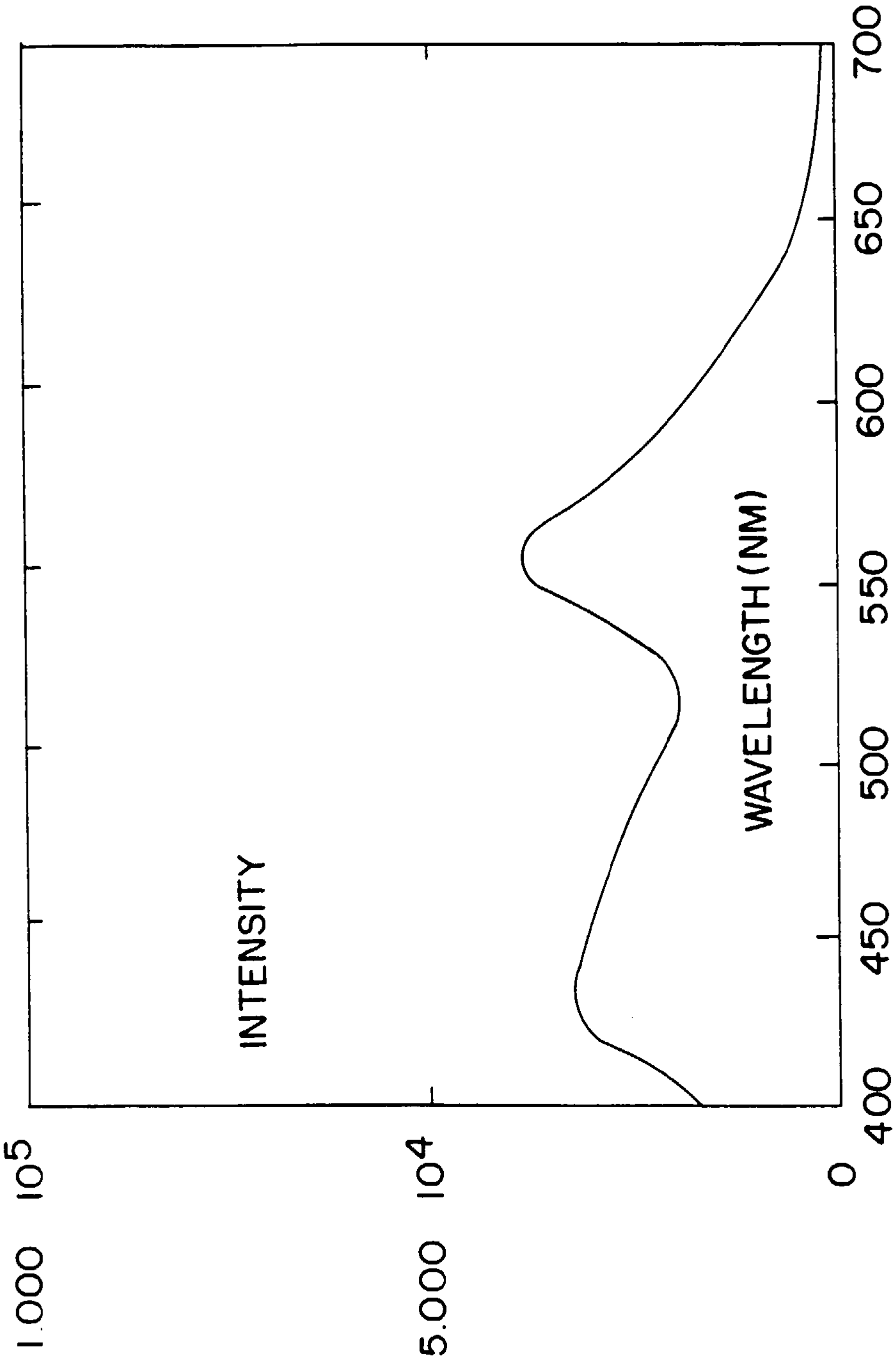
INTENSITY
WAVELENGTH (NM)
400
450
500
550
600
650
700
0
5.000 $10^4$
1.000 $10^5$

FIG. 8A

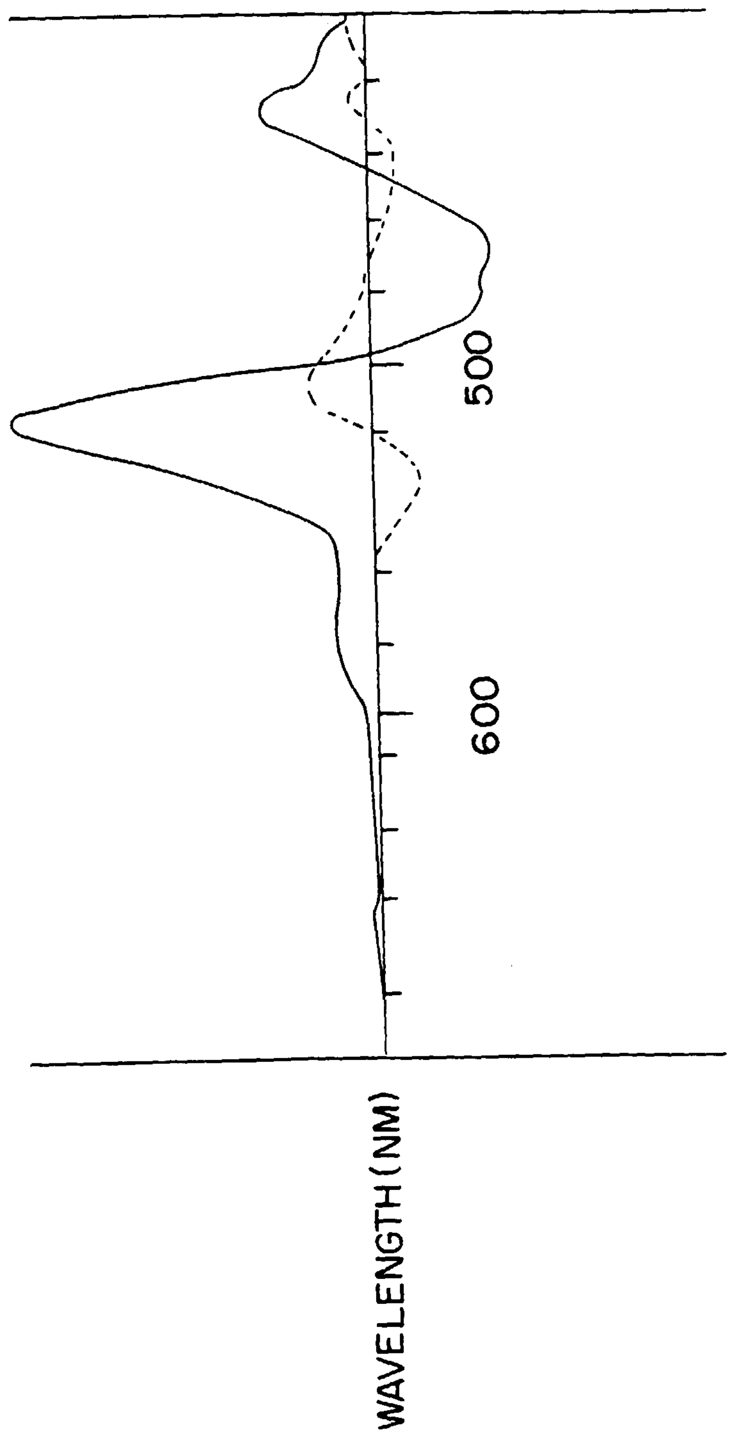

FIG. 8B

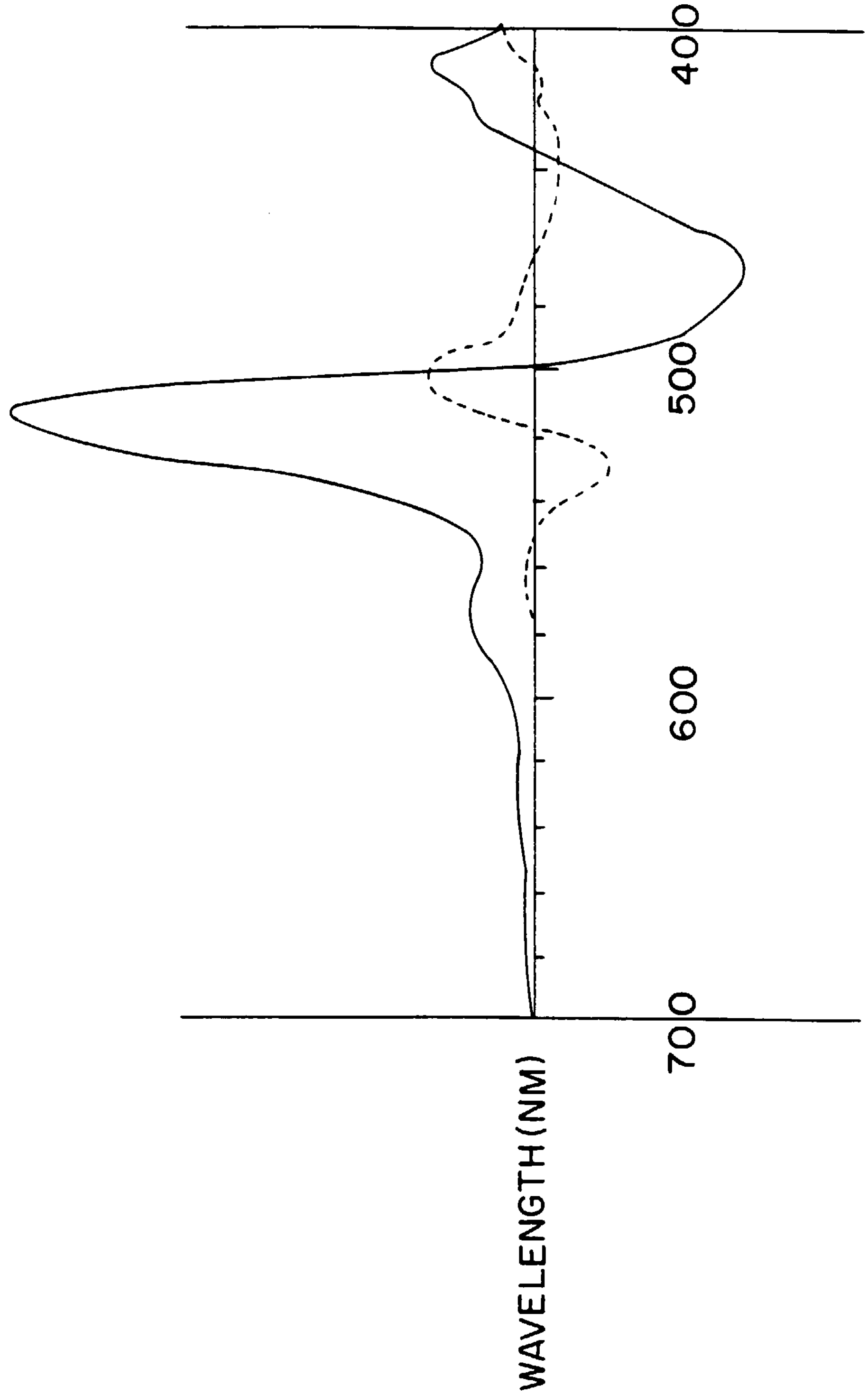

FIG. 8C

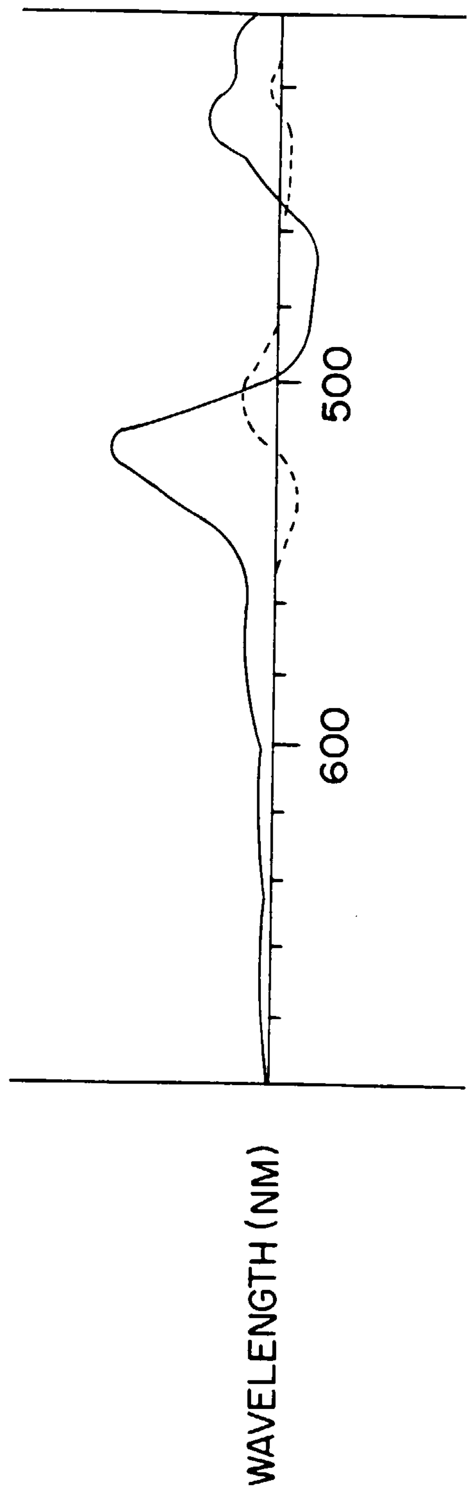

FIG.9A

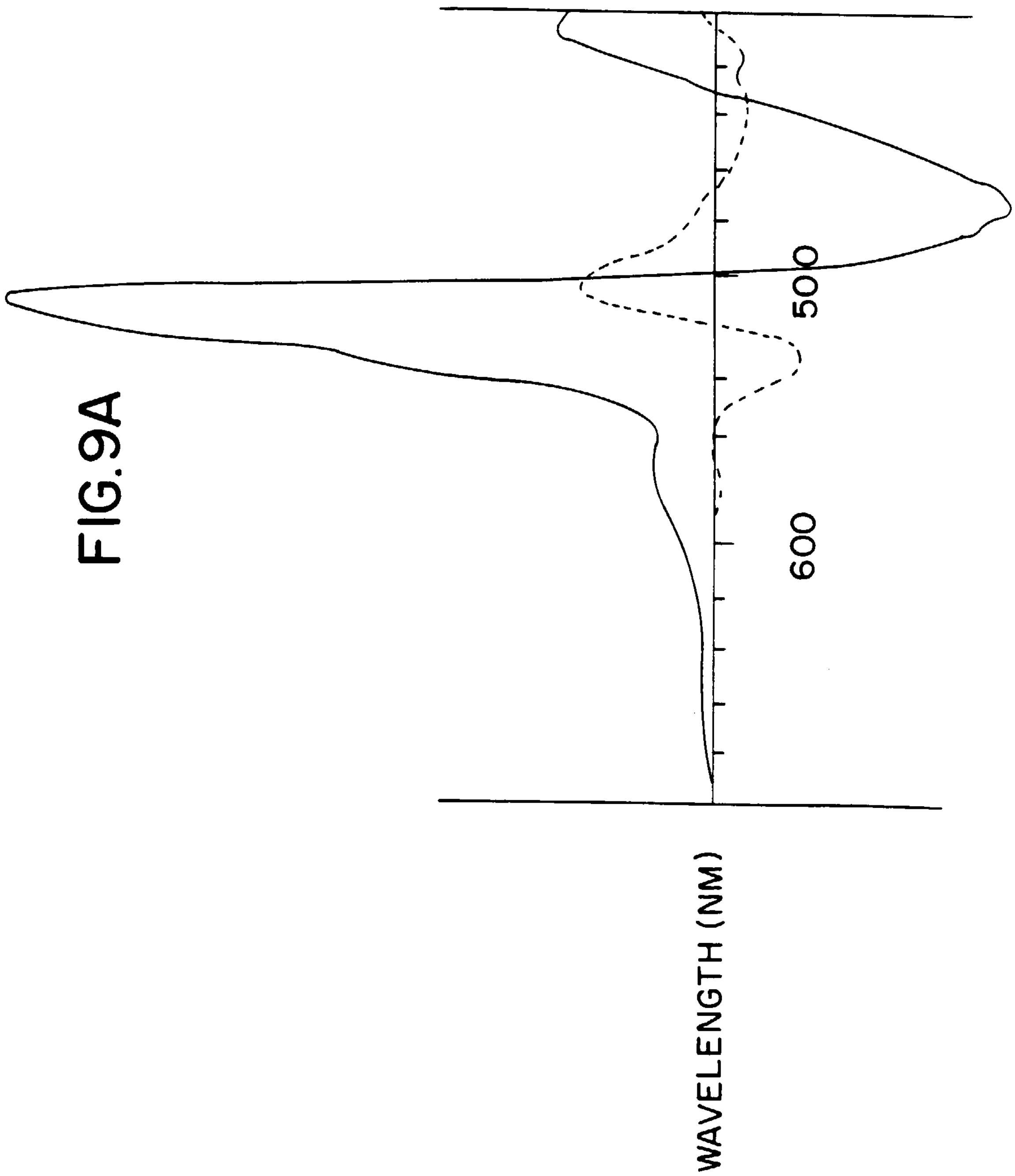

FIG. 9B

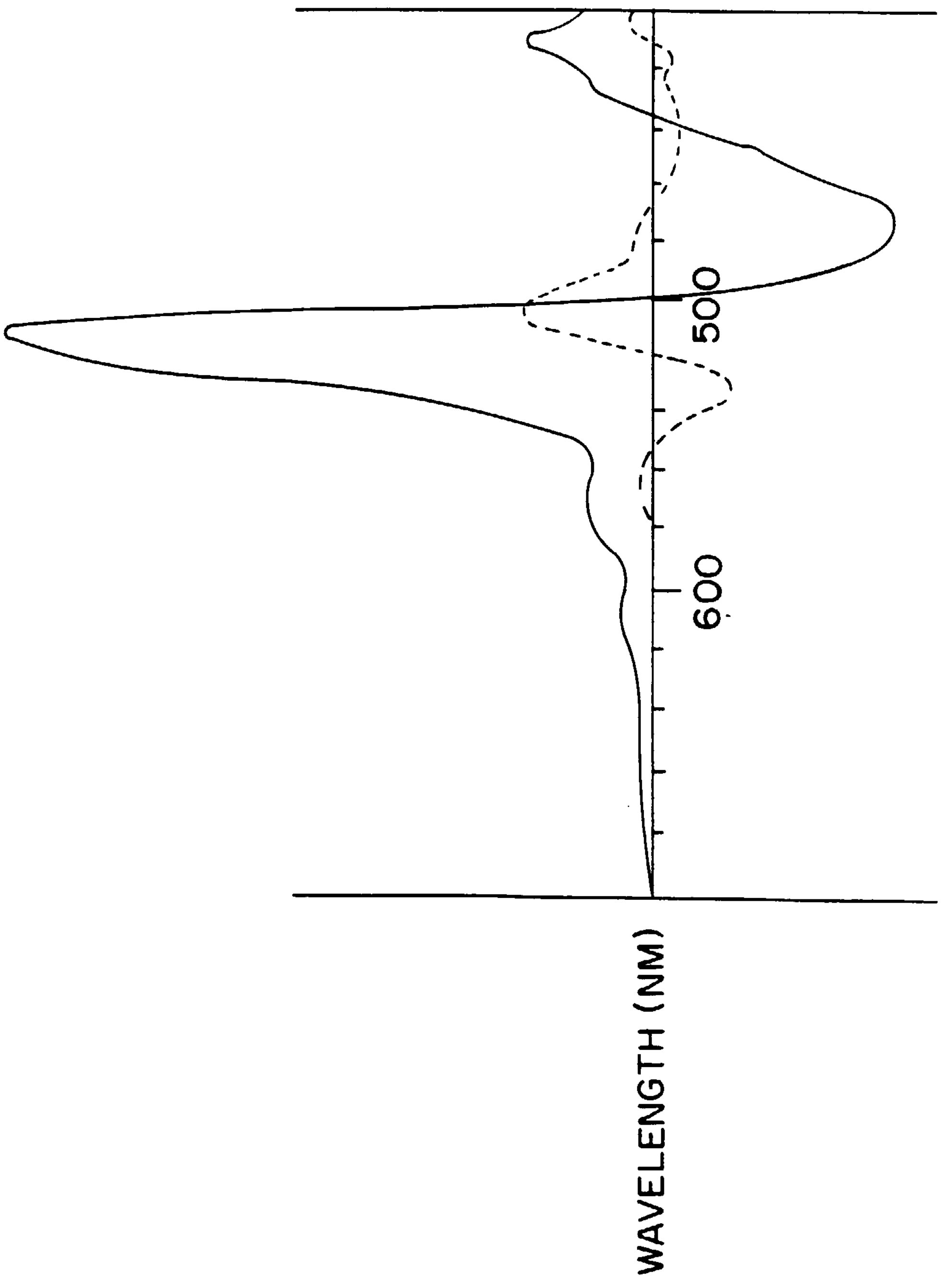

FIG. 10

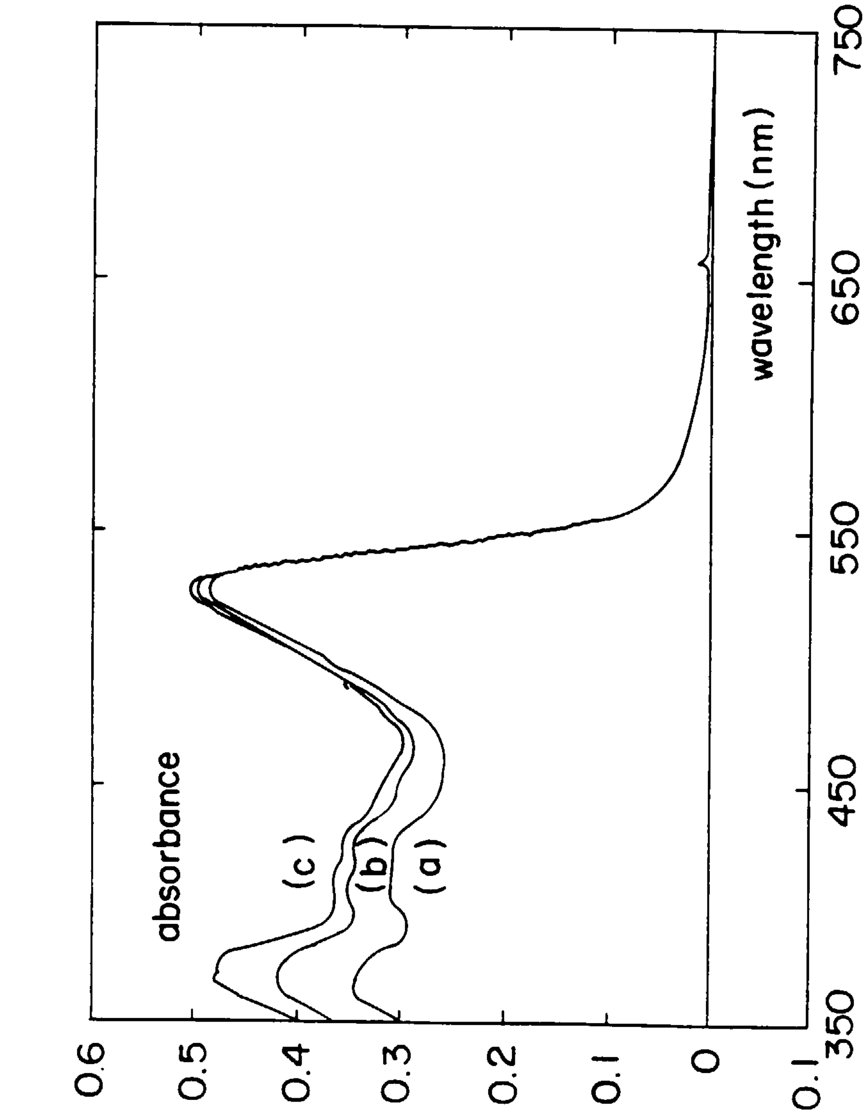
absorbance
0.6
0.5
0.4
0.3
0.2
0.1
0
-0.1
(a)
(b)
(c)
350
450
550
650
750
wavelength (nm)

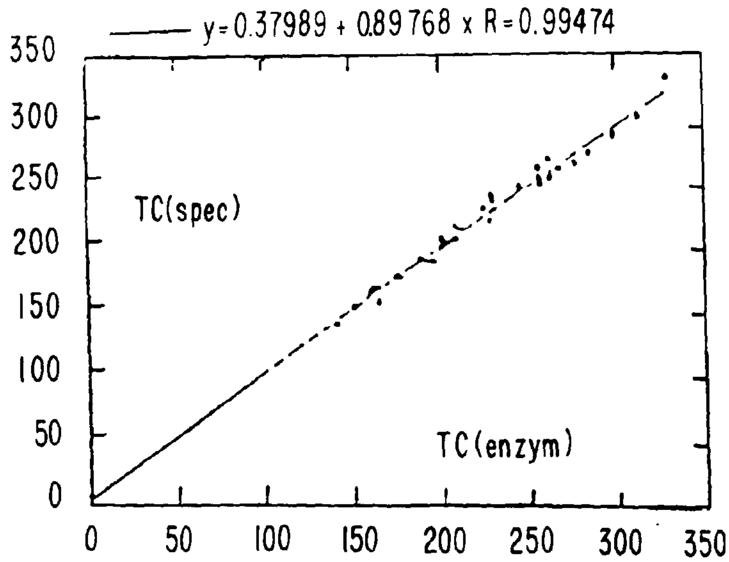


FIG. 11A

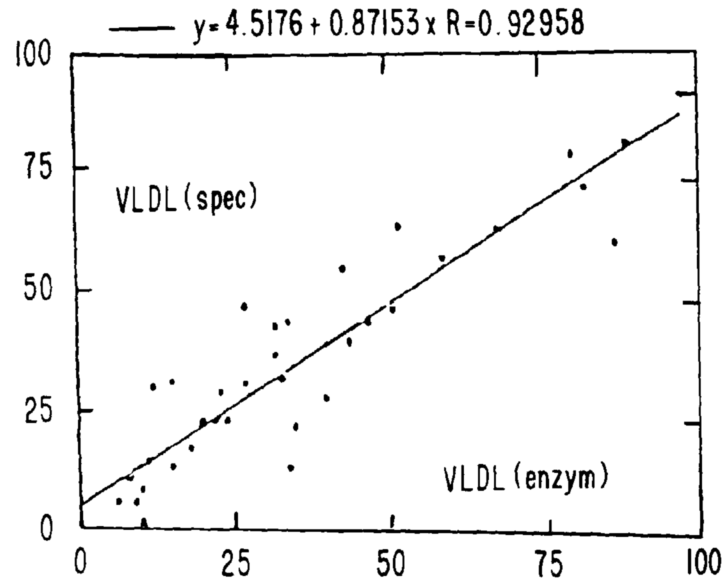


FIG. 11B

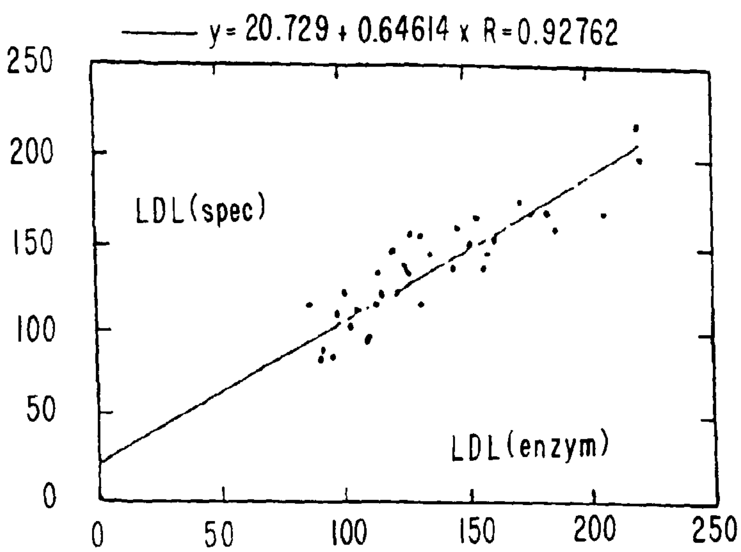


FIG. 11C

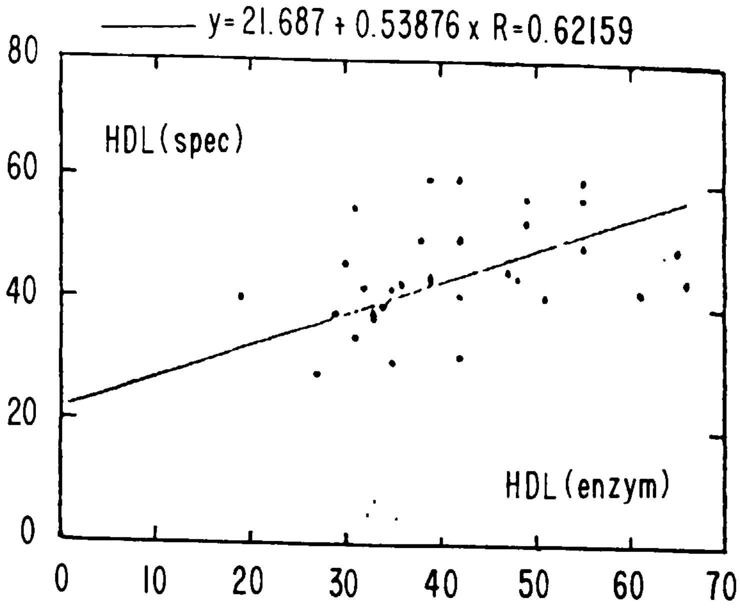


FIG. 12A

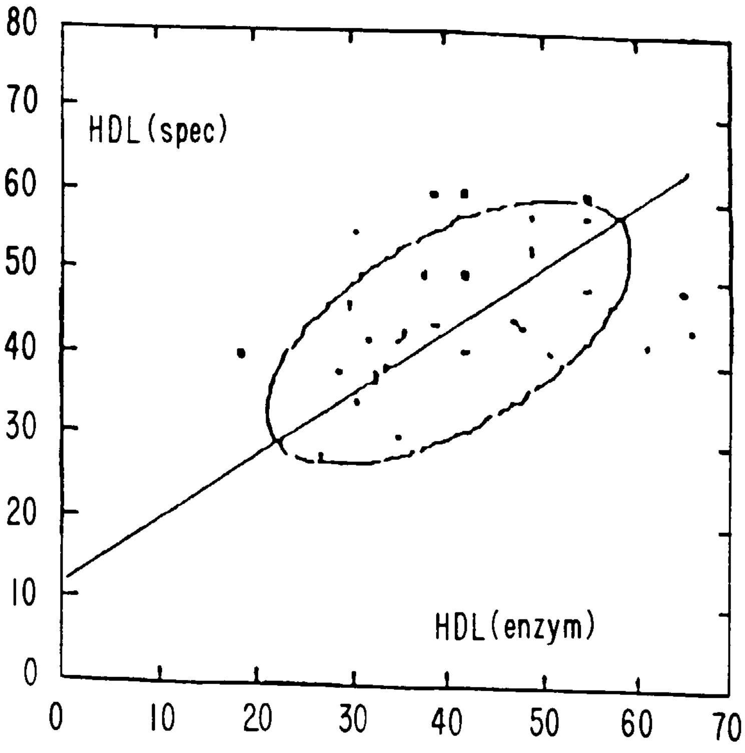


FIG. 12B

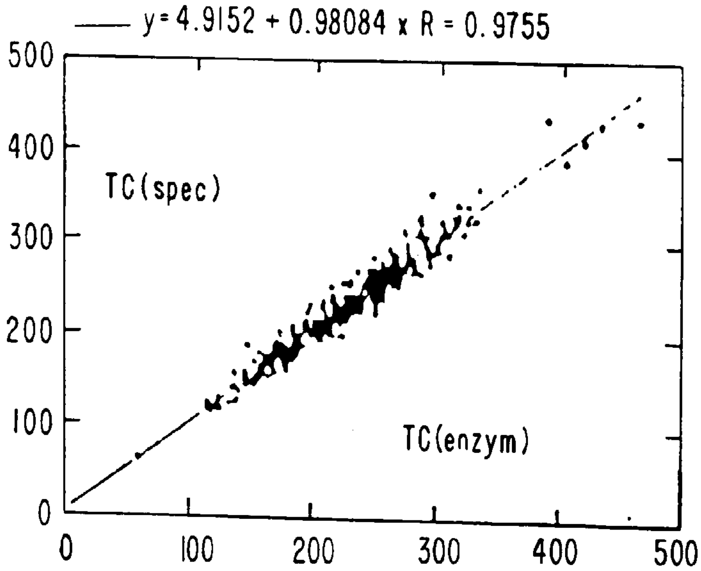


FIG. 13A

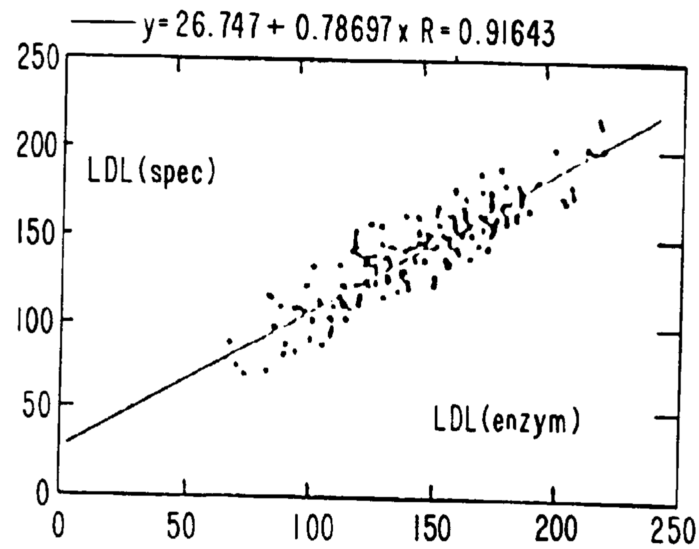


FIG. 13B

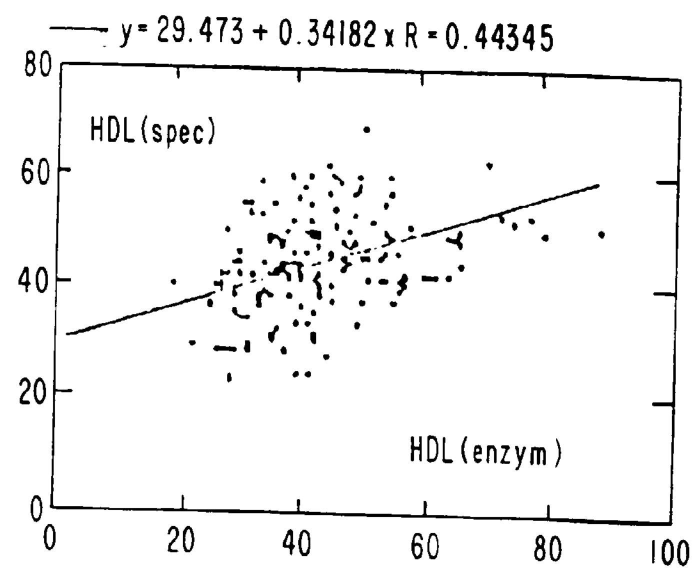


FIG. 13C

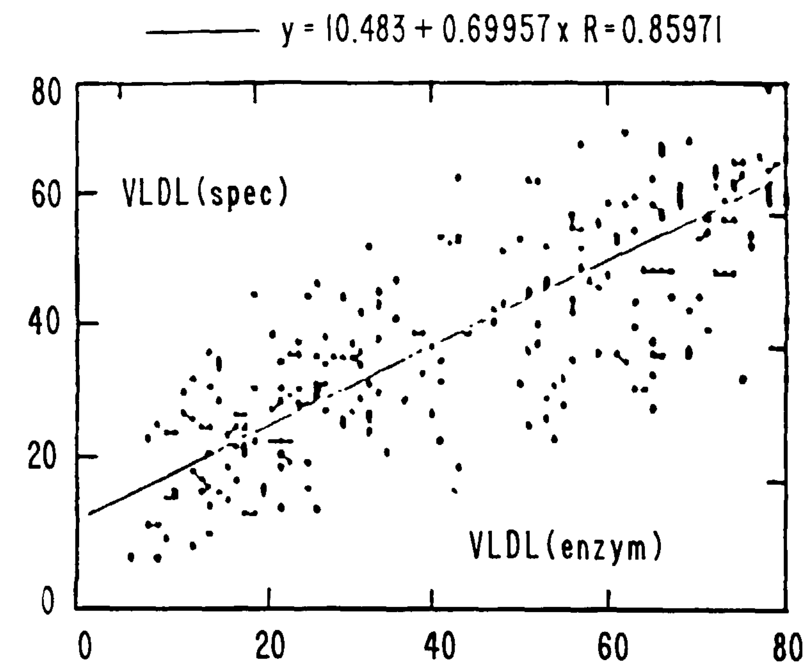


FIG. 14A

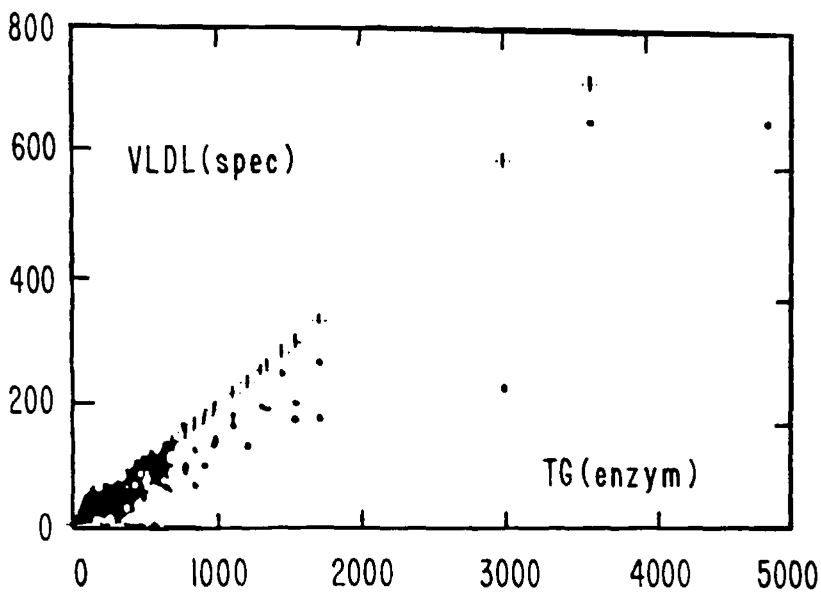


FIG. 14B

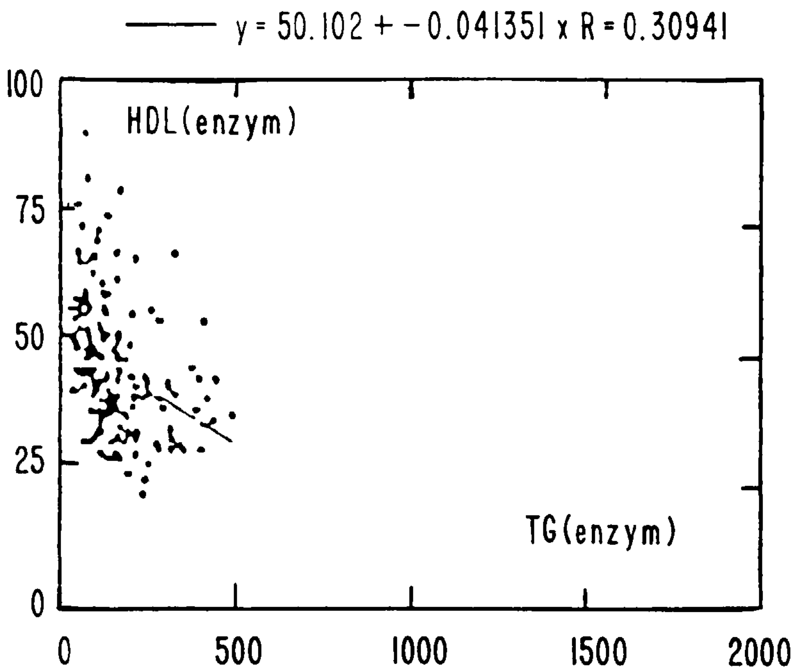


FIG. 15A

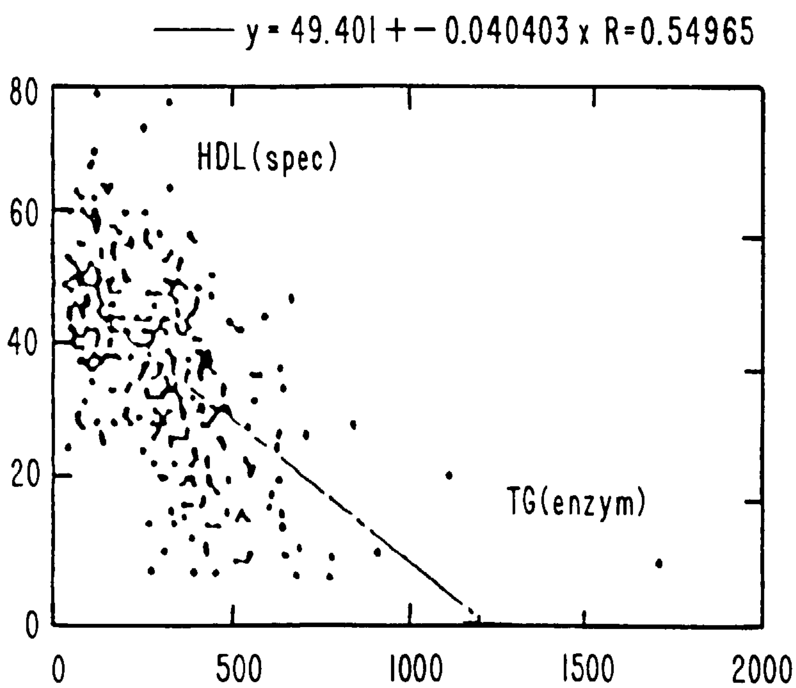


FIG. 15B